(19) Europäisches Patentamt
European Patent Office
Office européen des brevets

(11) EP 1 867 684 A1

(12) **EUROPEAN PATENT APPLICATION**
published in accordance with Art. 158(3) EPC

(43) Date of publication:
**19.12.2007 Bulletin 2007/51**

(21) Application number: **06731028.4**

(22) Date of filing: **04.04.2006**

(51) Int Cl.:
***C09B 31/08*** (2006.01) ***C07D 209/48*** (2006.01)
***C07D 215/38*** (2006.01) ***C07D 235/26*** (2006.01)
***C07D 277/66*** (2006.01) ***C09B 31/22*** (2006.01)
***C09D 201/00*** (2006.01) ***G02B 5/30*** (2006.01)

(86) International application number:
**PCT/JP2006/307080**

(87) International publication number:
**WO 2006/107035 (12.10.2006 Gazette 2006/41)**

(84) Designated Contracting States:
**DE**

(30) Priority: **04.04.2005 JP 2005107636**
**07.04.2005 JP 2005110535**
**21.04.2005 JP 2005123092**
**07.10.2005 JP 2005295499**
**27.03.2006 JP 2006084605**

(71) Applicant: **Mitsubishi Chemical Corporation**
**Minato-ku**
**Tokyo 108-0014 (JP)**

(72) Inventors:
- **SANO, H.,**
**Mitsubishi Chem. Grp Science & Tech.**
**Yokohama-shi,**
**Kanagawa 227-8502 (JP)**
- **YONEYAMA, T.,**
**Mitsubishi Chem. Grp Science & Tech.**
**Yokohama-shi,**
**Kanagawa 227-8502 (JP)**
- **NISHIMURA, M.,**
**Mitsubishi Chem. Grp Science & Tech**
**Yokohama-shi,**
**Kanagawa 227-8502 (JP)**
- **SHIMIZU, W.,**
**Mitsubishi Chem. Grp Science & Tech.**
**Yokohama-shi,**
**Kanagawa 227-8502 (JP)**
- **HASEGAWA, R.,**
**Mitsubishi Chem. Grp Science & Tech.**
**Yokohama-shi,**
**Kanagawa 227-8502 (JP)**
- **KADOWAKI, M.,**
**Mitsubishi Chem. Grp Science & Tech.**
**Yokohama-shi,**
**Kanagawa 227-8502 (JP)**
- **OIZUMI, J.,**
**Mitsubishi Chem. Grp Science & Tech.**
**Yokohama-shi,**
**Kanagawa 277-8502 (JP)**

(74) Representative: **Merkle, Gebhard**
**ter Meer Steinmeister & Partner GbR**
**Mauerkircherstrasse 45**
**D-81679 München (DE)**

(54) **COLORING MATTER FOR ANISOTROPIC COLORING MATTER FILM, COMPOSITION COMPRISING SAID COLORING MATTER, ANISOTROPIC COLORING MATTER FILM, AND POLARIZING ELEMENT**

(57) It is to provide a dye for an anisotropic dye film to be formed by a wet system film-forming method, which is achromatic and has high dichroism and a high degree of molecular orientation, an anisotropic dye film containing the dye and a polarizing element employing the film.

A dye for an anisotropic dye film to be formed by a wet system film-forming method, of which the free acid form is represented by the following formula (I), a composition for an anisotropic dye film containing the dye, an anisotropic dye film and a polarizing element employing the anisotropic dye film:

EP 1 867 684 A1

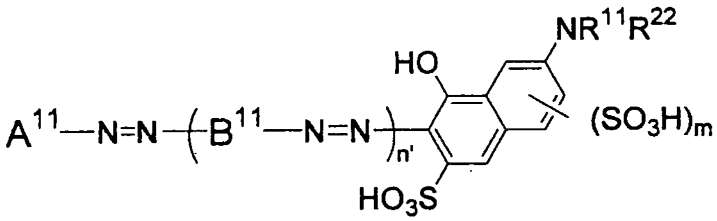

(I)

wherein $A^{11}$: a (substituted) phenyl group, a (substituted) naphthyl group or a (substituted) aromatic heterocyclic group;
$B^{11}$: a bivalent aromatic hydrocarbon group or aromatic hydrocyclic group;
$R^{11}$, $R^{22}$: H, OH, a (substituted) alkyl group, a (substituted) phenyl group or a (substituted) acyl group;
n': 1 or 2; m: 0 or 1.

## Description

### TECHNICAL FIELD

The present invention relates to dyes for anisotropic dye films showing high dichroism, which are useful for anisotropic dye films to be formed by a wet system film-forming method, particularly for e.g. polarizing plates provided on display devices such as light controlling devices, liquid crystal devices (LCD) and organic electroluminescence devices (OLED), and compositions containing the dyes, anisotropic dye films and polarizing elements.

### BACKGROUND ART

In LCD, linear polarizing plates or circular polarizing plates are used to control optical rotation or birefringence in display. Also in OLED, circular polarizing plates are used to prevent reflection of outside light. Heretofore, for such polarizing plates (polarizing elements), iodine has been widely used as a dichroic material. However, if iodine which is highly sublimable is used for a polarizing element, its heat resistance or light fastness is inadequate. Further, the extinction color becomes dark grayish blue, and an ideal achromatic color polarizing element for the entire visible spectral region cannot necessarily be obtained.

Therefore, a polarizing element has been studied wherein an organic dye is used as a dichroic material. However, such an organic dye has a problem such that only polarizing elements are obtainable which are substantially inferior in dichroism as compared with ones employing iodine.

Particularly in LCD employing as the display principle optical rotation or birefringence of light, a polarizing element is an important constituent, and a new polarizing element has been developed for the purpose of improving display performance and the like in recent years.

As one method, a method may be mentioned wherein, in the same manner as in the case of a polarizing element containing iodine, an organic dye having dichroism (dichroic dye) is resolved or adsorbed in a polymer material such as a polyvinyl alcohol, and the obtained film is stretched in one direction into a film so that the dichroic dye is oriented. However, this method has such a problem that the process such as stretching treatment is troublesome.

Therefore, another method has attracted attention in recent years. Non-Patent Document 1 discloses a method wherein a dichroic dye is oriented on a substrate such as glass or a transparent film utilizing e.g. intermolecular interaction of organic dye molecules to form a polarizing film (anisotropic dye film). However, the method disclosed in the document has been known to be problematic in heat resistance.

Further, the orientation of a dichroic dye on a substrate such as glass or a transparent film utilizing e.g. intermolecular interaction of organic dye molecules is achieved by a wet system film-forming method. In a case where an anisotropic dye film is prepared by such a wet system film-forming method, the dye to be used for the dye film is required not only to show high dichroism of the dye molecules but also to be a dye suitable for a process for the wet system film-forming method. The process for the wet system film-forming method may, for example, be a step of depositing and orienting the dye on a substrate and a step of controlling the orientation. Therefore, even a dye which can be used for a polarizing element by means of the above conventional stretching treatment may not be suitable for a wet system film-forming method in many cases.

Patent Documents 1 to 3 propose materials suitable for the above process. However, although such materials are suitable for the process, they have had such a drawback that they cannot show high dichroism.

Further, Patent Document 4 proposes as a material suitable for the process, a dye represented by (chromogen) $(SO_3M)n$. However, in Patent Document 4, several types of dichroic dyes are combined to achieve achromatic color, but when an anisotropic dye film is obtained by combining a several types of dichroic dyes in such a manner, the molecular orientation is likely to be disturbed as different molecules are mixed, and it tends to be difficult to obtain high dichroism.

Non-Patent Document: Dreyer, J.F., Journal de Physique, 1969, 4, 114., "Light Polarization From Films of Lyotropic Nematic Liquid Crystals"
Patent Document 1: JP-A-2002-180052
Patent Document 2: JP-A-2002-528758
Patent Document 3: JP-A-2002-338838
Patent Document 4: JP-A-8-511109

### DISCLOSURE OF THE INVENTION

### OBJECT TO BE ACCOMPLISHED BY THE INVENTION

The present invention is to provide a dye for an anisotropic dye film to be formed by a wet system film-forming

method, the film being achromatic, having high dichroism and a high degree of molecular orientation, and capable of being used to prepare a useful polarizing element.

MEANS TO ACCOMPLISH THE OBJECT

The present inventors have conducted extensive studies to accomplish the above object and as a result, they have found that by using a dye, of which the free acid form is represented by the following formula (I), an anisotropic dye film formed by a wet system film-forming method, which is achromatic and has high dichroism and a high degree of molecular orientation, can be obtained, and a polarizing element can be obtained by using the anisotropic dye film. The present invention has been accomplished on the basis of this discovery.

Namely, the present invention resides in a dye for an anisotropic dye film to be formed by a wet system film-forming method, of which the free acid form is represented by the following formula (I):

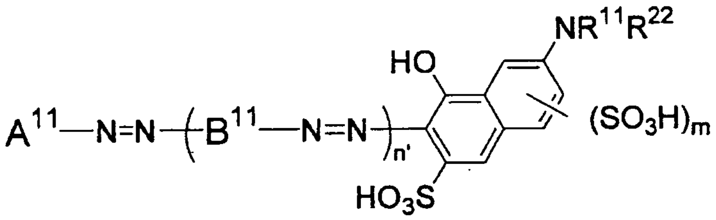

(I)

wherein each of $R^{11}$ and $R^{22}$ which are independent of each other, is a hydrogen atom, an alkyl group which may have a substituent, a phenyl group which may have a substituent, or an acyl group which may have a substituent; $A^{11}$ is a phenyl group which may have a substituent, a naphthyl group which may have a substituent, or an aromatic heterocyclic group which may have a substituent; $B^{11}$ is a bivalent aromatic hydrocarbon group which may have a substituent, or a bivalent aromatic heterocyclic group; m is 0 or 1; and n' is 1 or 2; provided that when n' is 1, $A^{11}$ is not a phenyl group having a vinyl group, and when n' is 2, $A^{11}$ is a group of the following formula (I-a) or (I-b) or an aromatic heterocyclic group which may have a substituent:

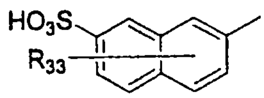

(I-a)

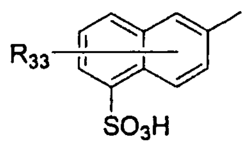

(I-b)

(wherein $R_{33}$ is a hydrogen atom, a hydroxy group or an alkoxy group which may have a substituent); provided that when n' is 2, a plurality of $B^{11}$ in one molecule may be the same or different; and a composition for an anisotropic dye film containing the dye, an anisotropic dye film and a polarizing element employing the anisotropic dye film.

The present invention further resides in an anisotropic dye film containing a dye, of which the free acid form is represented by the following formula (5), formed by a wet system film-forming method, and having a dichroic ratio of at least 40:

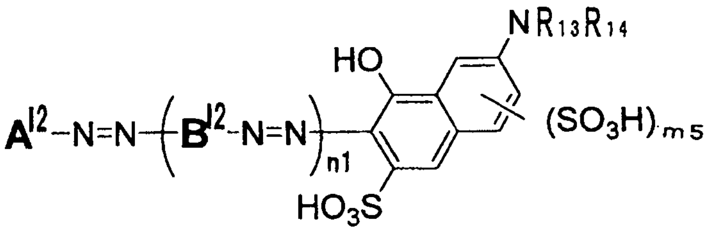

(5)

wherein $A^{12}$ is an aromatic hydrocarbon group which may have a substituent, or an aromatic heterocyclic group which may have a substituent; $B^{12}$ is a bivalent aromatic hydrocarbon group which may have a substituent, or a bivalent

aromatic heterocyclic group which may have a substituent; each of $R_{13}$ and $R_{14}$ which are independent of each other, is a hydrogen atom, an alkyl group which may have a substituent, a phenyl group which may have a substituent, or an acyl group which may have a substituent; $m^5$ is 0 or 1; and n1 is 1 or 2; provided that when n1 is 2, a plurality of $B^{12}$ in one molecule may be the same or different.

The present invention still further resides in an azo dye, of which the free acid form is represented by the following formula (6) :

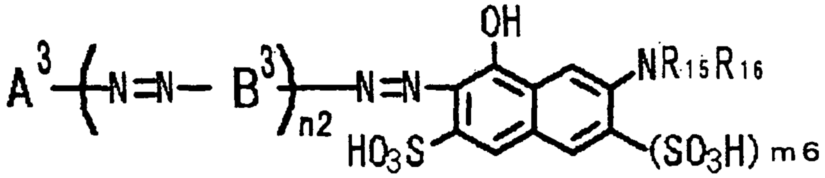
(6)

wherein $A^3$ is any of groups of the following formulae (6-a), (6-b) and (6-c), which may have a substituent:

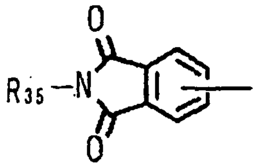
(6-a)

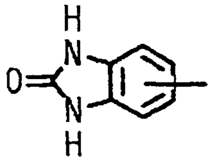
(6-b)

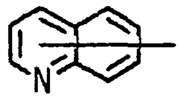
(6-c)

(wherein $R_{35}$ is a hydrogen atom, an alkyl group which may have a substituent, or a phenyl group which may have a substituent),

$B^3$ is a bivalent aromatic hydrocarbon group which may have a substituent, or a bivalent aromatic heterocyclic group containing as the hetero atom a nitrogen atom; each of $R_{15}$ and $R_{16}$ which are independent of each other, is a hydrogen atom, an alkyl group which may have a substituent, a phenyl group which may have a substituent, or an acyl group which may have a substituent; $m^6$ is 0 or 1, and n2 is 1 or 2;

provided that when n2 is 2, a plurality of $B^3$ in one molecule may be the same or different.

EFFECTS OF THE INVENTION

By using the dye of the present invention, an anisotropic dye film formed by a wet system film-forming method, which is achromatic and has high dichroism and a high degree of molecular orientation, can be provided. Further, a polarizing element employing an anisotropic dye film having such properties can be widely useful for e.g. display devices such as light control devices, liquid crystal devices and organic electroluminescence devices.

BRIEF DESCRIPTION OF THE DRAWING

Fig. 1 is a graph illustrating the relation between a dye concentration and a molar extinction coefficient (ε).

BEST MODE FOR CARRYING OUT THE INVENTION

The following description regarding constituting elements is one example (representative example) of the embodiments of the present invention, and the present invention is not limited thereto.

The anisotropic dye film in the present invention is a dye film having anisotropy in an electromagnetic characteristic in optional two directions selected from a total of three directions in a three-dimensional coordinate system comprising the thickness direction of the dye film and mutually perpendicular optional two in-plane directions.

The electromagnetic characteristic may, for example, be an optical characteristic such as absorption or refraction, or an electrical characteristic such as resistance or capacitance. A film having an optical anisotropy in e.g. absorption or refraction, may, for example, be a linearly polarizing film, a circularly polarizing film, a retardation film or an anisotropic electroconductive film.

The anisotropic dye film of the present invention is used preferably for a polarizing film, a retardation film or an anisotropic electroconductive film, more preferably for a polarizing film.

The present invention relates to a dye for an anisotropic dye film to be formed by a wet system film-forming method, of which the free acid form is represented by the following formula (I):

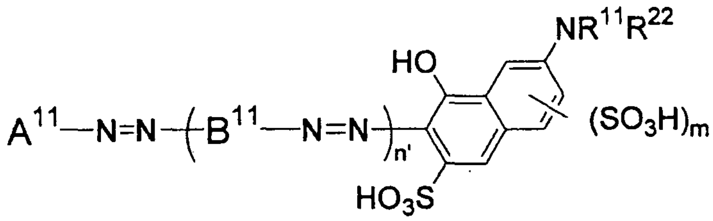

(I)

wherein each of $R^{11}$ and $R^{22}$ which are independent of each other, is a hydrogen atom, an alkyl group which may have a substituent, a phenyl group which may have a substituent, or an acyl group which may have a substituent; $A^{11}$ is a phenyl group which may have a substituent, a naphthyl group which may have a substituent, or an aromatic heterocyclic group which may have a substituent; $B^{11}$ is a bivalent aromatic hydrocarbon group which may have a substituent, or a bivalent aromatic heterocyclic group; m is 0 or 1, and n' is 1 or 2; provided that when n' is 1, $A^{11}$ is not a phenyl group having a vinyl group, and when n' is 2, $A^{11}$ is a group of the following formula (I-a) or (I-b) or an aromatic heterocyclic group which may have a substituent:

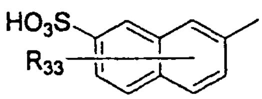

(I-a)

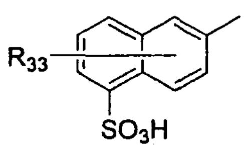

(I-b)

(wherein $R_{33}$ is a hydrogen atom, a hydroxy group or an alkoxy group which may have a substituent); provided that when n' is 2, a plurality of $B^{11}$ in one molecule may be the same or different.

In the present invention "which may have a substituent" means "which may have at least one substituent".

($A^{11}$)

$A^{11}$ is a phenyl group which may have a substituent, a naphthyl group which may have a substituent, or an aromatic heterocyclic group which may have a substituent.

The substituent which the phenyl group or the naphthyl group may have, is preferably a hydrophilic group to be introduced to increase the solubility of the azo compound, or an electron-donating group or an electron-withdrawing group to be introduced to adjust the chromaticness of the dye, and specifically, it may, for example, be an alkyl group which may have a substituent, an alkoxy group which may have a substituent, an acylamino group which may have a substituent, an amino group which may have a substituent, a carbamoyl group, a nitro group, a carboxy group, a sulfo group, a hydroxy group, a cyano group or a halogen atom.

The alkyl group has usually at least 1 and usually at most 6, preferably at most 4 carbon atoms. The group by which the alkyl group may be substituted may, for example, be an alkoxy group, a hydroxy group, a halogen atom, a sulfo group or a carboxy group. Specifically, the alkyl group may, for example, be a lower alkyl group which may have a substituent, such as a methyl group, an ethyl group, a n-propyl group, a hydroxyethyl group or a 1,2-dihydroxypropyl group.

The alkoxy group has usually at least 1 and usually at most 6, preferably at most 3 carbon atoms. The group by which the alkoxy group may be substituted may, for example, be an alkoxy group, a hydroxy group, a halogen atom, a sulfo group or a carboxy group. Specifically, the alkoxy group may, for example, be a lower alkoxy group which may have a substituent, such as a methoxy group, an ethoxy group, a n-propoxy group, a n-butoxy group, a hydroxyethoxy group or a 1,2-dihydroxypropoxy group.

The acylamino group is represented by -NH-COR$^{51}$, and R$^{51}$ is an alkyl group which may have a substituent, or a phenyl group which may have a substituent. The alkyl group has usually at least 1 and usually at most 4, preferably at most 2 carbon atoms. The group by which the alkyl group or the phenyl group may be substituted may, for example, be an alkoxy group, a hydroxy group, a sulfo group, a carboxy group or a halogen atom. Specifically, the acylamino group, may, for example, be an acetylamino group or a benzoylamino group.

The amino group is usually represented by -NH$_2$, -NHR$^{42}$ or -NR$^{43}$R$^{44}$, and each of R$^{42}$ to R$^{44}$ which are independent of one another, is an alkyl group which may have a substituent or a phenyl group which may have a substituent. The alkyl group has usually at least 1 and usually at most 4, preferably at most 2 carbon atoms. The group by which the alkyl group or the phenyl group may be substituted may, for example, be an alkoxy group, a hydroxy group, a sulfo group, a carboxy group or a halogen atom. Specifically, the amino group may, for example, be a methylamino group, an ethylamino group, a propylamino group, a dimethylamino group or a phenylamino group.

The carbamoyl group is not substituted, or is an alkylcarbamoyl group which may be substituted, a phenylcarbamoyl group which may be substituted or a naphthylcarbamoyl group which may be substituted. The alkyl group, the phenyl group or the naphthyl group in this substituent may have a substituent, and the group by which the alkyl group, the phenyl group or the naphthyl group may be substituted may, for example, be an alkoxy group, a hydroxy group, a sulfo group, a carboxy group or a halogen atom. Specifically, the carbamoyl group may, for example, be a carbamoyl group, a phenylcarbamoyl group or a naphthylcarbamoyl group.

The phenyl group or the naphthyl group may have 1 to 5 such substituents, and preferably has 1 to 2 substituents.

The aromatic heterocyclic group is preferably a group derived from a monocyclic or bicyclic heterocyclic ring. The atom constituting the aromatic heterocyclic group other than carbon atoms may be a nitrogen atom, a sulfur atom or an oxygen atom. In a case where the aromatic heterocyclic group has a plurality of atoms constituting the ring other than carbon atoms, they may be the same or different. Specifically, the aromatic heterocyclic group may, for example, be a pyridyl group, a quinolyl group, a thiazolyl group, a benzothiazolyl group, a quinolonyl group, a naphthalimidoyl group or the following groups:

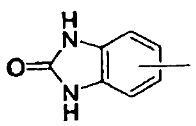 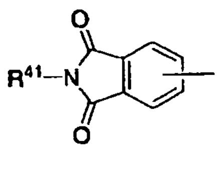 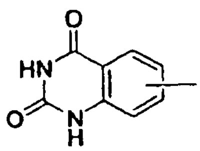

wherein R$^{41}$ is a hydrogen atom, an alkyl group which may have a substituent or a phenyl group which may have a substituent; and the substituent may, for example, be an alkyl group such as a methyl group or an ethyl group, an alkoxy group such as a methoxy group or an ethoxy group, a hydroxy group, a nitro group, a sulfo group, a carboxy group, a halogen atom, an amino group such as an amino group or a methylamino group, an amide group or a cyano group.

Among them, a pyridyl group, a quinolyl group or a phthalimidoyl group is preferred.

The substituent which the aromatic heterocyclic group may have may, for example, be an alkyl group such as a methyl group or an ethyl group, an alkoxy group such as a methoxy group or an ethoxy group, a hydroxy group, a nitro group, a sulfo group, a carboxy group, a halogen atom, an amino group such as an amino group or a methylamino group, an amide group or a cyano group. Among them, preferably the aromatic heterocyclic group is not substituted or is substituted by a hydroxy group, a sulfo group or a carboxy group.

In the dye represented by the formula (I), A$^{11}$ is preferably an aromatic heterocyclic group, whereby association properties of the dye will improve, and such a dye is suitable for both anisotropic dye film to be formed by a wet system film-forming method and anisotropic dye film to be formed by a dry system film-forming method (stretching method).

($B^{11}$)

$B^{11}$ is a bivalent aromatic hydrocarbon group which may have a substituent, or a bivalent aromatic heterocyclic group which may have a substituent.

The aromatic hydrocarbon group is preferably a phenylene group or a naphthylene group. The substituent which the aromatic hydrocarbon group may have may, for example, be an alkyl group which may have a substituent, an alkoxy group which may have a substituent, a hydroxy group, a nitro group, a sulfo group, a carboxy group, a halogen atom, an amino group which may have a substituent, an acylamino group which may have a substituent or a cyano group. With respect to the alkyl group which may have a substituent, the alkoxy group which may have a substituent, the amino group which may have a substituent and the acylamino group which may have a substituent, their preferred number of carbon atoms, examples of substituents which they may have, and their specific examples are as defined for the above case where $A^{11}$ is a phenyl group or a naphthyl group. Among them, preferred is a hydrogen bond-forming group or a group having low polarity such as an alkyl group, an alkoxy group, a hydroxy group or a halogen atom, in view of improvement in association properties by the interaction in formation of lyotropic liquid crystals, and from the viewpoint of water solubility, preferred is a sulfo group. The aromatic hydrocarbon group may be non-substituted or may have from 1 to 5 such substituents, and preferably has from 1 to 2 substituents.

The aromatic heterocyclic group is preferably a group derived from a monocyclic or bicyclic heterocyclic ring. The atom constituting the aromatic heterocyclic group other than carbon atoms, may be a nitrogen atom, a sulfur atom or an oxygen atom, and a nitrogen atom is particularly preferred. In a case where the aromatic heterocyclic group has a plurality of atoms constituting the ring other than carbon, they may be the same or different. Specifically, the aromatic heterocyclic group may, for example, be a pyridinediyl group, a quinolinediyl group, an isoquinolinediyl group, a benzothiadiazolediyl group or a phthalimidediyl group. Among them, a quinolinediyl group or an isoquinolinediyl group is preferred.

The substituent which the aromatic heterocyclic group may have may, for example, be an alkyl group such as a methyl group or an ethyl group, an alkoxy group such as a methoxy group or an ethoxy group, an amino group such as a non-substituted amino group or a methylamino group, an acetylamino group, an acylamino group, a nitro group, a carboxy group, a sulfo group, a hydroxy group, a cyano group or a halogen atom. Among them, a hydroxy group, a sulfo group or a carboxy group is preferred. The aromatic heterocyclic group may be non-substituted or may have from 1 to 5 such substituents, and preferably it is non-substituted or has from 1 to 2 substituents.

($R^{11}$ and $R^{22}$)

Each of $R^{11}$ and $R^{22}$ which are independent of each other, is a hydrogen atom, an alkyl group which may have a substituent, a phenyl group which may have a substituent, or an acyl group which may have a substituent. The substituent which the alkyl group, the phenyl group or the acyl group may have may be a hydroxy group, a carboxy group or a sulfo group. The acyl group may be an alkylacyl which may be substituted or a phenylacyl which may be substituted, and the substituent which the alkyl group or the phenyl group may have may be a hydroxy group, a carboxy group or a sulfo group.

Preferably, both $R^{11}$ and $R^{22}$ are hydrogen atoms.

Specific examples of the dye represented by the formula (I) in the free acid form include dyes of the formulae (1) to (5) as described hereinafter, and further include the following dyes:

(1-21)

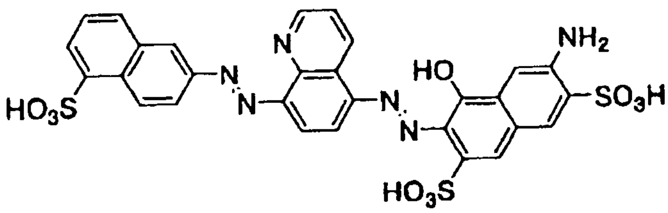

(1-22)

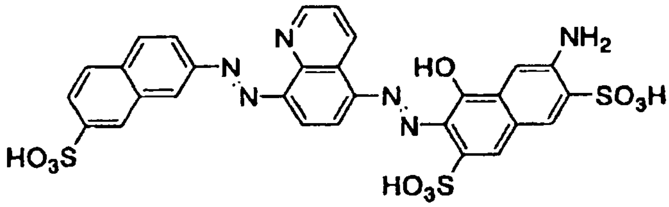

(1-24)

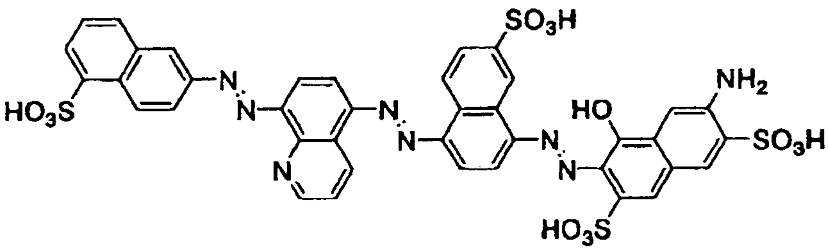

(3-34)

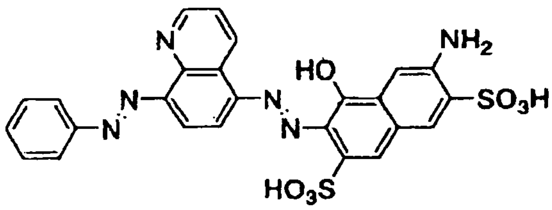

(4-24)

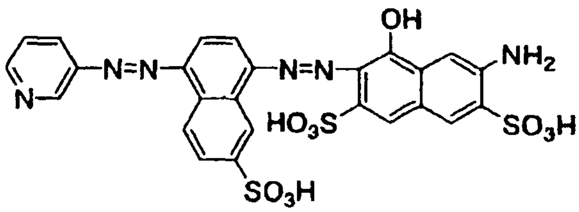

(4-25)

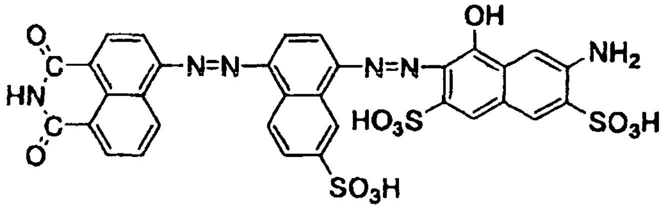

(4-27)

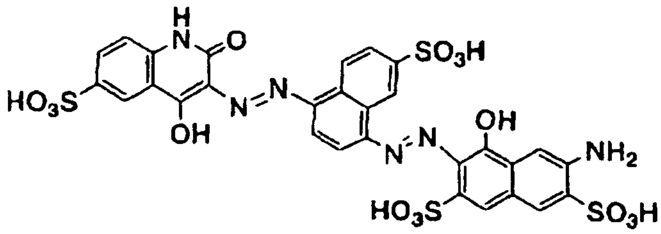

(4-28)

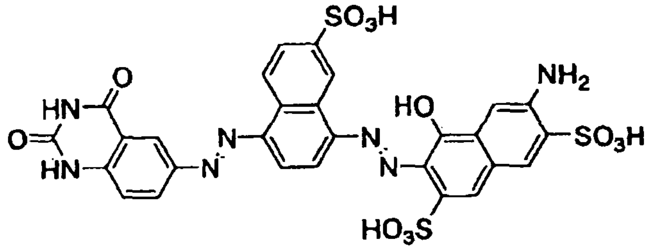

The dye of the present invention is a dye for an anisotropic dye film to be formed by a wet system film-forming method, of which the free acid form is represented by the above formula (I), and more specifically, it may, for example, be a water soluble black dichroic azo dye represented by the following formula (1) or (2):

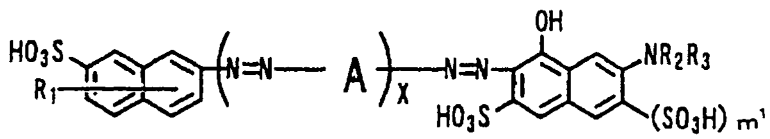 (1)

wherein A is a phenylene group which may have a substituent, or a naphthylene group which may have a substituent; $R_1$ is a hydrogen atom, a hydroxy group or an alkoxy group which may have a substituent; each of $R_2$ and $R_3$ which are independent of each other, is a hydrogen atom, an alkyl group which may have a substituent, a phenyl group which may have a substituent, or an acyl group which may have a substituent; $m^1$ is 0 or 1; and X is 1 or 2; provided that when X is 2, a plurality of A in one molecule may be the same or different;

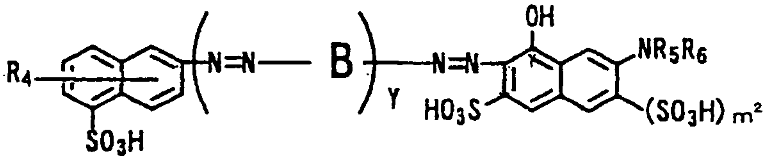 (2)

wherein B is a phenylene group which may have a substituent, or a naphthylene group which may have a substituent; $R_4$ is a hydrogen atom, a hydroxy group or an alkoxy group which may have a substituent; each of $R_5$ and $R_6$ is a hydrogen atom, an alkyl group which may have a substituent, a phenyl group which may have a substituent, or an acyl group which may have a substituent; $m^2$ is 0 or 1; and Y is 1 or 2; provided that when Y is 2, a plurality of B in one molecule may be the same or different.

The azo dye represented by the formula (1) or (2), as evident from its molecular structure, has naphthalene rings on both ends of the molecular major axis and has such a structure that substituents which impart strong attractive force to other molecules are disposed at specific positions on the naphthalene rings. Accordingly, the respective molecules have an interaction due to planarity, and the respective molecules are likely to be in an association state.

The reasons why the molecules of the dye of the present invention are likely to be in an association state are considered to be as follows:

(i) It is considered that since the respective dye molecules have substituents which impart strong attractive force to other molecules on both ends of the molecular major axis, they are attracted to each other and likely to be in an association state.

(ii) It is considered that since the respective molecules have naphthalene rings on both ends, the molecules having high planarity are attracted to each other and likely to be in an association state.

(iii) It is considered that since the substituents which impart strong attractive force to other molecules are at the specific positions on both ends of the molecular major axis (a naphthyl group having e.g. a sulfo group at the 7- or 5-position on one end and a naphthyl group having a (substituted) amino group at the 7-position on the other end), the sulfo group or the like at the 7- or 5-position and the amino group at the 7-position can get closer to each other due to the positional relationship at the time of salt forming, whereby they are strongly attracted to each other and likely to be in a stable association state.

It is considered that the molecules of the dye of the present invention are likely to be in an association state due to the above three factors (i) to (iii), whereby a high lyotropic liquid crystalline state will be achieved.

Further, not only the azo dye represented by the formula (1) or (2) of the present invention is black, but also a composition containing this dye can provide a high degree of molecular orientation state by means of a process specific to the wet system film-forming method i.e. a lamination process by i.e. coating on the surface of a substrate. This means that it is possible to form an achromatic dye film with high anisotropy.

Heretofore, when it is attempted to obtain an achromatic anisotropic dye film by using one dichroic dye, the molecular orientation is likely to be disturbed by the steric repulsion of substituents introduced to the dye molecules, and it has been difficult to obtain high dichroism. Accordingly, in the case of a conventional wet system film-forming method, an achromatic anisotropic dye film is obtained by combination of a plural types of dyes in many cases. However, the dye of the present invention has a specific dye structure as described above, and thus forms a highly lyotropic liquid crystalline state and provides a high degree of molecular orientation state, and it is possible to provide black color only with one dye. Accordingly, an anisotropic dye film containing the dye of the present invention can function as an anisotropic dye film having high dichroism.

Now, the azo dyes represented by the above formulae (1) and (2) of the present invention will be described below.

(A and B)

In the above formulae (1) and (2), each of A and B which are independent of each other, is a phenylene group or a naphthylene group, which may have a substituent.

In the above formula (1), in a case where X is 2, two A in one molecule may be the same or different. Further, in the above formula (2), in a case where Y is 2, two B in one molecule may the same or different.

The phenylene group is preferably a 1,4-penylene group, and the naphthylene group is preferably a 1,4-naphthylene group, with a view to obtaining the above interaction.

The substituent which the phenylene group may have is preferably a hydrogen bond-forming group or a group having low polarity such as an alkyl group (preferably a $C_{1-4}$ alkyl group (such as a methyl group, an ethyl group, a n-propyl group, a hydroxyethyl group or a 1,2-dihydroxypropyl group)) which may have a substituent, an alkoxy group (preferably a $C_{1-4}$ alkoxy group (such as a methoxy group, an ethoxy group, a n-propoxy group, a n-butoxy group, a hydroxyethoxy group or a 1,2-dihydroxypropoxy group)) which may have a substituent, or an acylamino group (preferably a $C_{2-7}$ acylamino group (such as an acetylamino group or a benzoylamino group)) which may have a substituent, in view of improvement in association properties by the interaction in formation of lyotropic liquid crystals.

The substituent of the phenylene group may further have a substituent, and specifically, it may be those exemplified as substituents of the phenylene group, or a hydroxy group or a halogen atom.

The substituent which the naphthylene group may have is preferably a hydroxy group, a sulfo group, or an alkoxy group (preferably a $C_{1-4}$ alkoxy group (such as a methoxy group, an ethoxy group, a hydroxyethoxy group or a 1,2-dihydroxypropoxy group)) which may have a substituent, in view of improvement in association properties by the interaction in formation of lyotropic liquid crystals.

The substituent which the alkoxy group may have may be a hydroxy group or an alkoxy group.

($R_1$ and $R_4$)

In the above formulae (1) and (2), each of $R_1$ and $R_4$ which are independent of each other, is a hydrogen atom, a hydroxy group or an alkoxy group (preferably a $C_{1-3}$ alkoxy group (such as a methoxy group, an ethoxy group, a

hydroxyethoxy group or a 1,2-dihydroxypropoxy group)) which may have a substituent.

($R_2$, $R_3$, $R_5$ and $R_6$)

In the above formulae (1) and (2), each of $R_2$, $R_3$, $R_5$ and $R_6$ which are independent of one another, is a hydrogen atom, an alkyl group (preferably a $C_{1-4}$ alkyl group (such as a methyl group or an ethyl group)) which may have a substituent, a phenyl group which may have a substituent, or an acyl group (such as an acetyl group or a benzoyl group) which may have a substituent. The substituent which the alkyl group, the phenyl group or the acyl group may have may be a hydroxy group, a carboxy group or a sulfo group.

Particularly preferably, in the formula (1), either $R_2$ or $R_3$ is a hydrogen atom, and in the formula (2), either $R_5$ or $R_6$ is a hydrogen atom.

(X and Y)

Each of X and Y which are independent of each other, is 1 or 2.

($m^1$ and $m^2$)

Each of $m^1$ and $m^2$ which are independent of each other, is 0 or 1.

(Molecular weight)

The molecular weight of the dye represented by the above formula (1) or (2) is usually at least 650 and usually at most 1,500, preferably at most 1,100, in the free acid form.

In the dye represented by the above formula (1) or (2), in the dye structure, the molecule has naphthyl groups on both ends of the molecular major axis, and the substituents of the naphthyl groups on both ends and the substitution positions (a naphthyl group having a substituent at the 7- or 5-position and a naphthyl group having an amino group at the 7-position) are specified, whereby association properties are improved, and a high lyotropic liquid crystalline state can be formed, as described above. Accordingly, the dye represented by the above formula (1) or (2) of the present invention is suitable as the dye for an anisotropic dye film to be formed by a wet system film-forming method. Further, since it has a high dichroic ratio, by using a dye composition employing the dye, an anisotropic dye film having high dichroism can be obtained.

Specific examples of the dye of the present invention include dyes having structures of the following formulae (1-1) to (1-20), (1-23), and (1-25) to (1-27) in the free acid form. However, the dye of the present invention is not limited thereto:

(1-1)

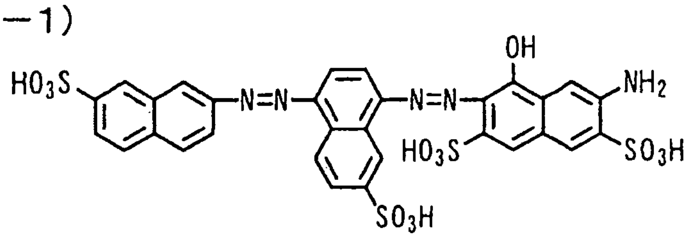

(1-2)

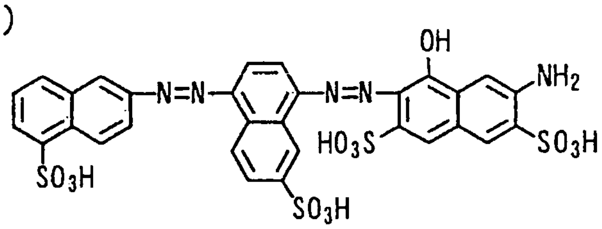

(1-3)

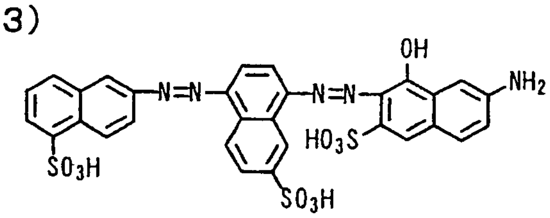

(1-4)

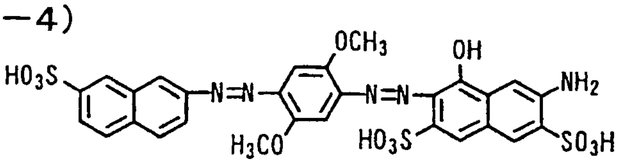

(1-5)

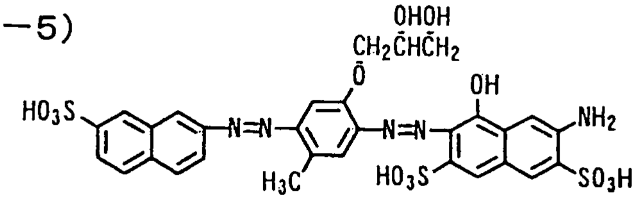

(1-6)

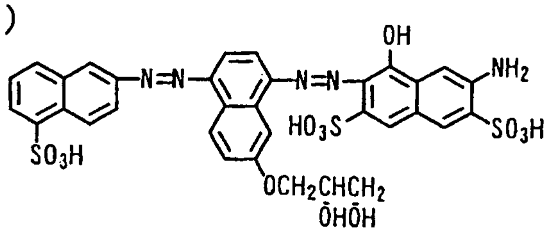

(1-7)

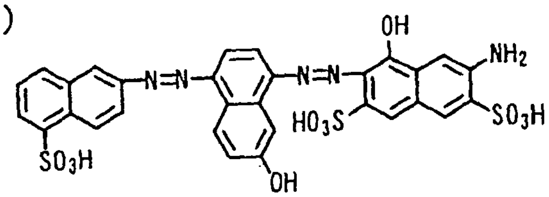

(1-8)

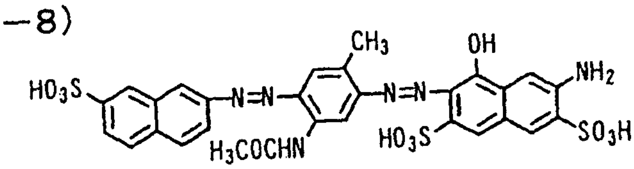

(1-9)

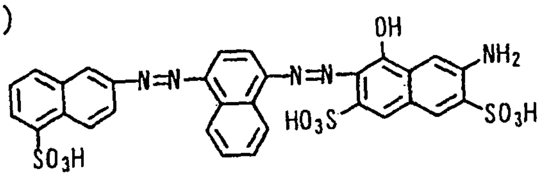

(1-10)

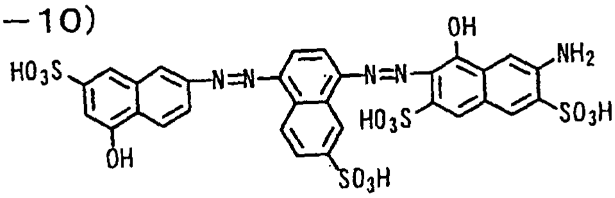

(1-11)

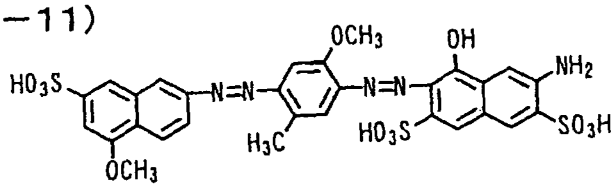

(1-12)

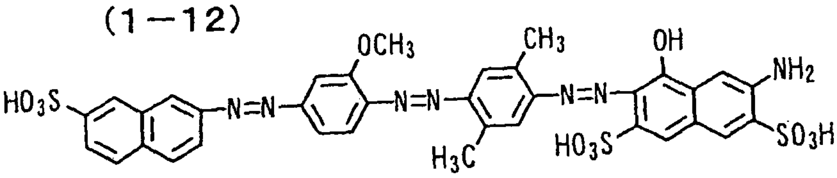

(1-13)

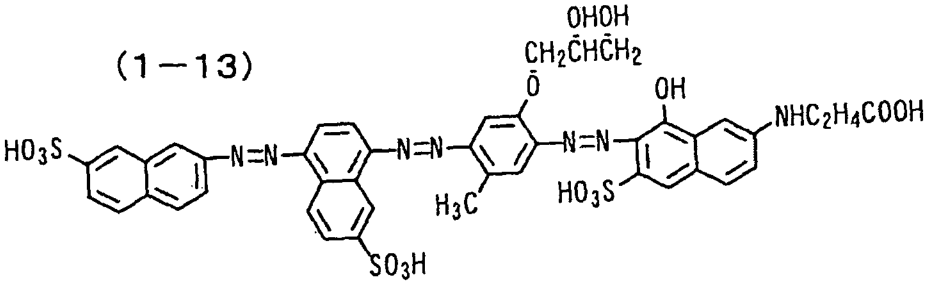

(1-14)

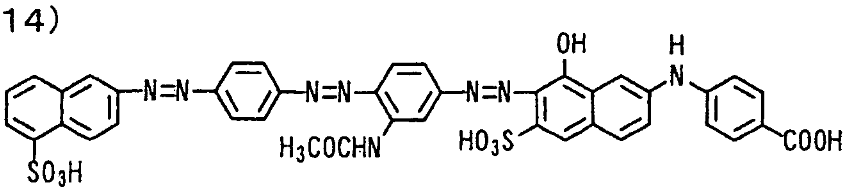

(1－15)

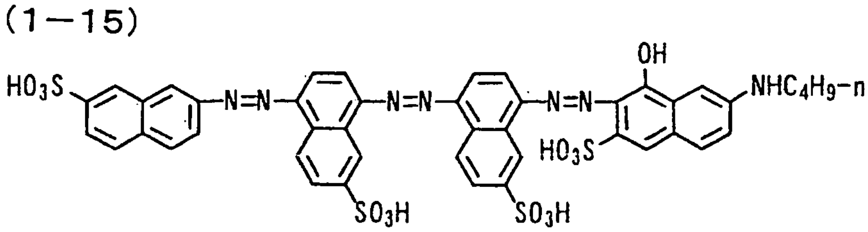

(1－16)

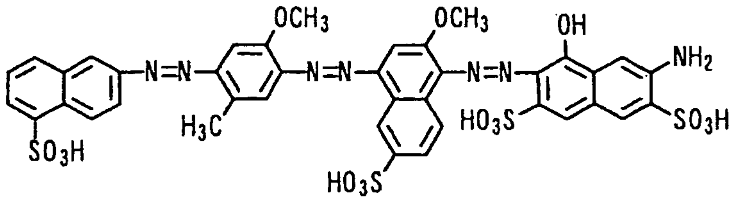

(1－17)

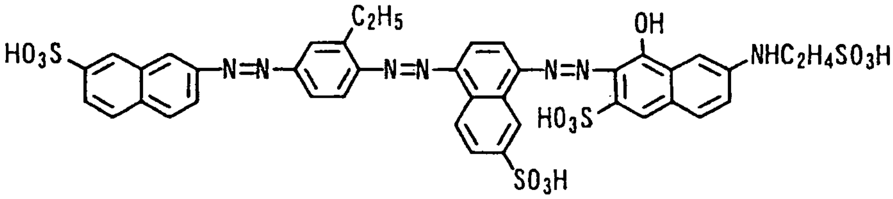

(1－18)

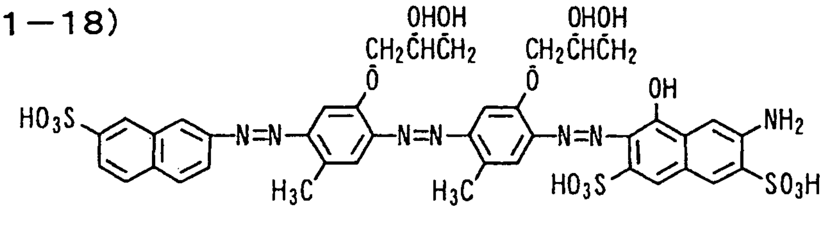

(1－19)

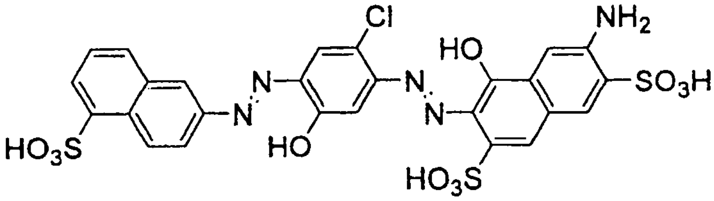

(1-20)

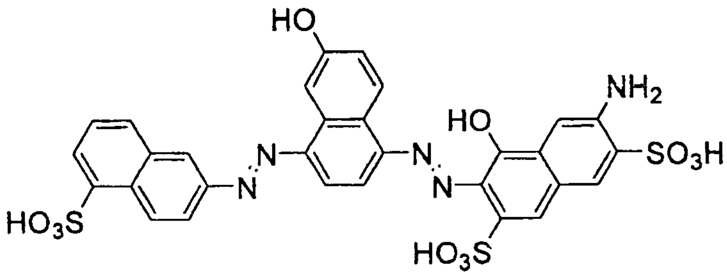

(1-23)

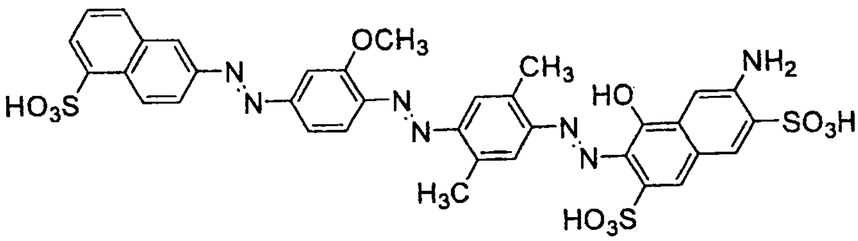

(1-25)

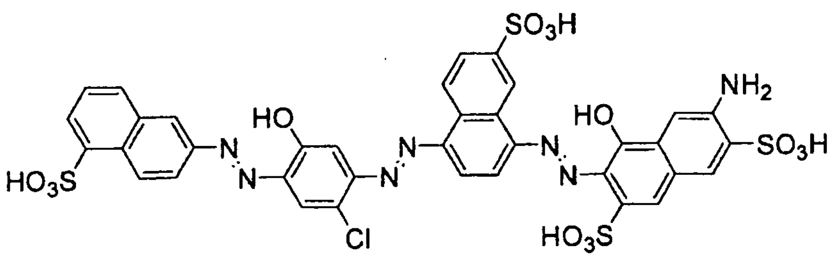

(1-26)

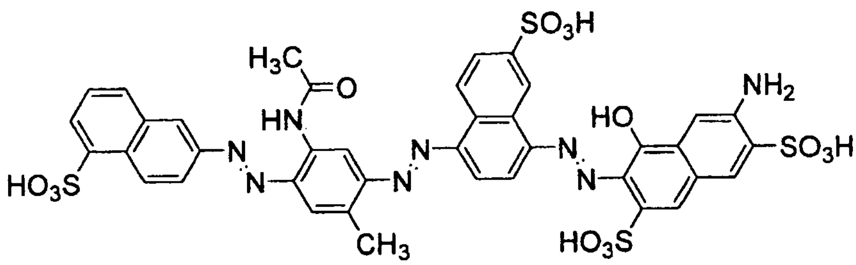

(1-27)

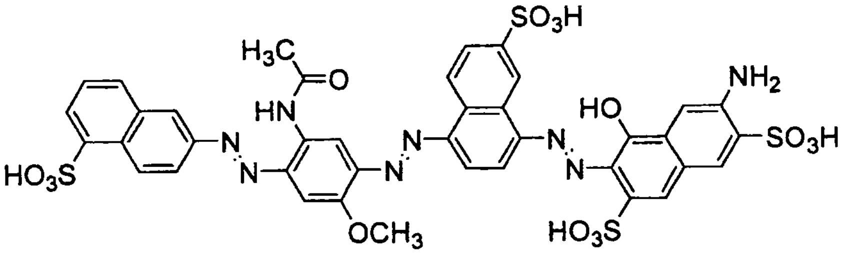

The azo dye represented by the above formula (1) or (2) may be produced in accordance with a known method. For example, dye No. (1-1) may be produced in accordance with the following steps (a1) and (b1).

(a1) In accordance with a conventional method (such as "Shin Senryo Kagaku (New Dye Chemical)", Yutaka Hosoda (published on December 21, 1973, GIHODO SHUPPAN Co., Ltd.), pages 396-409), a monoazo compound is produced from 7-amino-2-naphthalenesulfonic acid (Delta acid) and 8-amino-2-naphthalenesulfonic acid (1,7-Cleves acid) by means of diazotization and coupling.
(b1) The obtained monoazo compound is subjected to diazotization and coupling reaction with 7-amino-1-naphthol-3,6-disulfonic acid (RR acid) in accordance with a conventional method in the same manner, and salting out is carried out with sodium chloride to obtain a desired dye No. (1-1).

Particularly, the dye represented by the above formula (1-1) of the present invention forms lyotropic liquid crystals in the aqueous solution, and accordingly, an anisotropic dye film having high dichroism can be prepared with it, and it is a useful dye particularly suitable for the wet system film-forming method.

As another specific example of the azo dye which is the dye for an anisotropic dye film, of which the free acid from in represented by the above formula (I) of the present invention, a water soluble black dichroic azo dye represented by the following formula (3) may be mentioned:

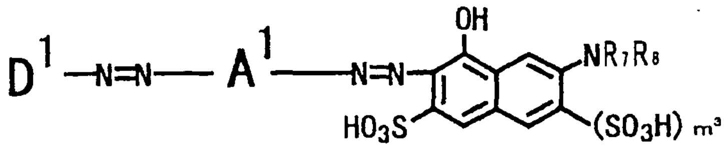 (3)

wherein $D^1$ is a phenyl group which may have a substituent other than a vinyl group, a naphthyl group which may have a substituent, or an aromatic heterocyclic group which may have a substituent; $A^1$ is an aromatic hydrocarbon group which may have a substituent; each of $R_7$ and $R_8$ which are independent of each other, is a hydrogen atom, an alkyl group which may have a substituent, a phenyl group which may have a substituent, or an acyl group which may have a substituent; and $m^3$ is 0 or 1.

The dye represented by the above formula (3) of the present invention is usually a water soluble dye, and is usually a dichroic dye, depending upon the number of hydrophilic groups in the molecule.

($D^1$)

In the formula (3), $D^1$ is a phenyl group which may have a substituent other than a vinyl group, a naphthyl group which may have a substituent, an aromatic heterocyclic group which may have a substituent. Among them, preferred is a phenyl group which may have a substituent or a naphthyl group which may have a substituent, and particularly preferred is a phenyl group which may have a substituent, in view of both liquid crystallinity and solubility.

Particularly, the substituent of $D^1$ is preferably a group having polarity. The group having polarity may be an ionic substituent such as a carboxy group or a sulfo group, a substituent having a hydrogen bond-forming proton such as a hydroxy group, an amino group, a hydroxyethyl group, a 1,2-dihydroxypropyl group, an acylamino group or a carbamoyl group, or a substituent having high polarity and containing an atom (such as a nitrogen atom, an oxygen atom or a sulfur atom) with high electronegativity, such as an alkoxy group, a cyano group or a dialkylamino group.

In a case where $D^1$ is a phenyl group, the phenyl group may have a substituent other than a vinyl group. The vinyl group includes a vinyl group and substituted vinyl groups such as a vinylene group and a vinylidene group.

The substituent which the phenyl group may have is preferably a hydrophilic group to be introduced to increase the solubility of the dye, or an electron-donating group or an electron-withdrawing group to be introduced to adjust the chromaticness. Specifically, it may, for example, be an alkyl group (preferably a $C_{1-4}$ alkyl group) which may be substituted, such as a methyl group, an ethyl group, a n-propyl group, a hydroxyethyl group or a 1,2-dihydroxypropyl group; or an alkoxy group (preferably a $C_{1-4}$ alkoxy group) which may be substituted, such as a methoxy group, an ethoxy group, a n-propoxy group, a n-butoxy group, a hydroxyethoxy group or a 1,2-dihydroxypropoxy group.

Further, it may, for example, be an amino group which may be substituted, such as an alkylamino group (preferably an amino group substituted by $C_{1-4}$ alkyl group) such as a methylamino group, an ethylamino group, a propylamino group or a dimethylamino group, a phenylamino group, or an acylamino group (preferably an amino group substituted by $C_{2-7}$ acyl group) such as an acetylamino group or a benzoylamino group; a carbamoyl group which may be substituted, such as a phenylaminocarbonyl group or a naphthylaminocarbonyl group; a carboxy group; a sulfo group; a hydroxy group; a phenyl group; an aromatic heterocyclic group such as a benzothiazolyl group, a quinolyl group or a phthalimidoyl group; a halogen atom such as fluorine, chlorine or bromine; a nitro group; or a cyano group. Among these substituents, preferred is a sulfo group, a hydroxy group, a carboxy group, a cyano group, a carbamoyl group, a methyl group, a methoxy group or a chlorine atom.

The above substituent may further have a substituent, and the substituent may, for example, be a hydroxy group, a sulfo group, an alkoxy group, an alkyl group, a cyano group, a nitro group or a halogen atom.

The phenyl group may have from 1 to 5 such substituents, and preferably has from 1 to 2 substituents.

In a case where $D^1$ is a naphthyl group, the naphthyl group may have a substituent. The substituent which the naphthyl group may have is preferably a hydrophilic group to be introduced to increase the solubility, or an electron-donating group or an electron-withdrawing group to be introduced to adjust the chromaticness. Specifically, it may be the same group as the substituents which the above phenyl group may have, and it may be an alkyl group (preferably a $C_{1-4}$ alkyl group) which may be substituted, an alkoxy group (preferably a $C_{1-4}$ alkoxy group) which may be substituted, an amino group which may be substituted (preferably an amino group which may be substituted by a $C_{1-7}$ alkyl or alkoxy group or a $C_{2-7}$ acyl group), a carboxy group, a sulfo group, a hydroxy group, or a cyano group. The substituent of the alkyl group, the alkoxy group or the amino group may be those exemplified as the substituents of the naphthyl group.

The naphthyl group may have from 1 to 4 such substituents, and preferably has from 1 to 2 substituents. Further, among the above substituents, preferred is a sulfo group, a hydroxy group or a carboxy group.

In a case where $D^1$ is a naphthyl group, it may be a 1-naphthyl group, a 2-naphthyl group or a 3-naphthyl group, and preferred is a 2-naphthyl group or a 3-naphthyl group with a view to lowering the concentration at which liquid crystallinity is obtained.

In a case where $D^1$ is a 1-naphthyl group, the naphthyl group preferably has a substituent at the 3-, 4-, 6- or 8-position of the naphthyl group with a view to developing liquid crystallinity, and particularly preferably it has a sulfo group, a carboxy group or a cyano group. In the formula (3), in a case where $D^1$ is a 3,6-disulfo-8-hydroxynaphthyl group, such a combination is excluded that $R_7$ and $R_8$ are hydrogen atoms and $m^3=0$.

In a case where $D^1$ is a 2-naphthyl group, the naphthyl group preferably has a substituent at the 1-, 4-, 5-, 6-, 7- or 8-position with a view to developing liquid crystallinity, and particularly preferably it has a substituent at the 5- or 7-position. Particularly preferably it has a sulfo group.

In a case where $D^1$ is a 3-naphthyl group, the naphthyl group preferably has a substituent at the 6-position with a view to developing liquid crystallinity, particularly preferably it has a sulfo group.

In a case where $D^1$ an aromatic heterocyclic group, the aromatic heterocyclic group may have a substituent. The hetero atom in the aromatic heterocyclic group may, for example, be a nitrogen atom or a sulfur atom, and preferred is an aromatic heterocyclic group having a nitrogen atom with a view to lowering the concentration at which liquid crystallinity is developed. Specifically, the aromatic heterocyclic group may, for example, be a pyridyl group, a quinolyl group, a thiazolyl group or a benzothiazolyl group, and it is preferably a pyridyl group.

The substituent which the aromatic heterocyclic group may have is preferably a hydrophilic group to be introduced to increase the solubility, or an electron-donating group or an electron-withdrawing group to be introduced to adjust the chromaticness. Specifically, it may, for example, be a sulfo group, a carboxy group, an alkyl group (preferably a $C_{1-4}$ alkyl group) which may have a substituent, an alkoxy group (preferably a $C_{1-4}$ alkoxy group) which may have a substituent, an amino group which may be substituted (preferably an amino group which may be substituted by a $C_{1-7}$ alkyl or alkoxy group), or a cyano group. The substituent of the alkyl group, the alkoxy group or the amino group may be those exemplified as the substituents of the aromatic heterocyclic group.

The aromatic heterocyclic group may have from 1 to 4 such substituents, and it preferably has from 1 to 2 substituents. Further, among the above substituents, preferred is a sulfo group or a carboxy group.

($A^1$)

In the above formula (3), $A^1$ is an aromatic hydrocarbon group which may have a substituent. Specifically, the aromatic hydrocarbon group may be a phenylene group or a naphthylene group.

The phenylene group is preferably a 1,4-phenylene group, and the naphthylene group is preferably a 1,4-naphthylene group, with a view to obtaining the above interaction.

In a case where $A^1$ is a phenylene group, the substituent which the phenylene group may have is preferably a group having low polarity, or a hydrogen bond-forming group, in view of improvement in association properties by the interaction in formation of lyotropic liquid crystals.

Specifically, it may, for example, be an alkyl group (preferably a $C_{1-4}$ alkyl group) which may be substituted, such as a methyl group, an ethyl group, a n-propyl group, a hydroxyethyl group or a 1,2-dihydroxypropyl group; an alkoxy group (preferably a $C_{1-4}$ alkoxy group) which may be substituted, such as a methoxy group, an ethoxy group, a n-propoxy group, a n-butoxy group, a hydroxyethoxy group or a 1,2-dihydroxypropoxy group; or an amino group which may be substituted, such as an acylamino group (preferably an amino group substituted by a $C_{2-7}$ acyl group) such as an acetylamino group or a benzoylamino group.

The phenylene group may have from 1 to 4 such substituents, and preferably has from 1 to 2 substituents.

The substituent of the phenylene group may further have a substituent, and specifically, it may, for example, be those exemplified as the substituents of the phenylene group or a hydroxy group.

In a case where $A^1$ is a naphthylene group, the substituent which the naphthylene group may have is preferably a hydroxy group, a sulfo group, an alkoxy group (preferably a $C_{1-4}$ alkoxy group (such as a methoxy group, an ethoxy group, a hydroxyethoxy group or a 1,2-dihydroxypropoxy group)) which may have a substituent, in view of improvement in association properties by interaction in formation of lyotropic liquid crystals.

The substituent of the naphthylene group may further have a substituent, and specifically, it may, for example, be those exemplified as substituents of the naphthylene group or a hydroxy group.

The naphthylene group may have from 1 to 4 such substituents, and preferably has from 1 to 2 substituents.

($R_7$ and $R_8$)

In the above formula (3), each of $R_7$ and $R_8$ which are independent of each other, is a hydrogen atom, an alkyl group which may have a substituent, a phenyl group which may have a substituent, or an acyl group which may have a substituent. Specifically, it may be an alkyl group (preferably a $C_{1-4}$ alkyl group) such as a methyl group or an ethyl group, a phenyl group, or an acyl group such as an acetyl group or a benzoyl group. The substituent which the alkyl group, the phenyl group or the acyl group may have may, for example, be a hydroxy group, a carboxy group or a sulfo group.

Particularly preferably, in the formula (3), at least one of $R_7$ and $R_8$ is a hydrogen atom.

In the above formula (3), $m^3$ is 0 or 1.

The molecular weight of the dye represented by the above formula (3) of the present invention is usually at least 450 and usually at most 1,500, preferably at most 1,100, in a free acid form.

The dye represented by the above formula (3) usually provides black color and can form a highly lyotropic liquid crystalline state. Therefore, the dye represented by the above formula (3) of the present invention is suitable as a dye for an anisotropic dye film to be formed by a wet system film-forming method. Further, it has low wavelength dispersion properties and has a high dichroic ratio, whereby an anisotropic dye film having a high degree of molecular orientation can be prepared by employing the dye. Accordingly, by using a dye composition employing the dye, an anisotropic dye film having high polarization characteristics can be obtained.

Specific examples of the dye represented by the formula (3) of the present invention in the free acid form include dyes having structures represented by the following formulae (3-1) to (3-33) and (3-35) to (3-59). However, the dye is not limited thereto:

(3−1)

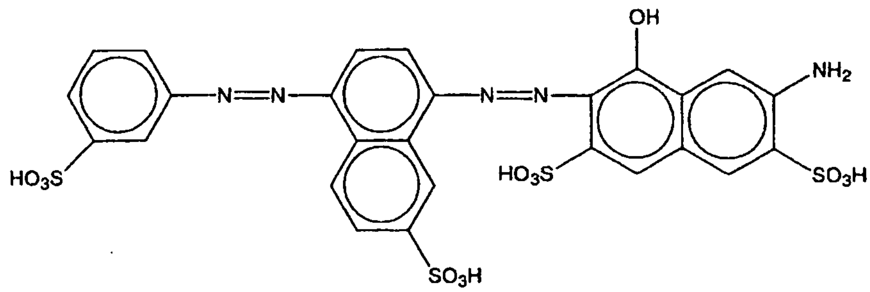

(3−2)

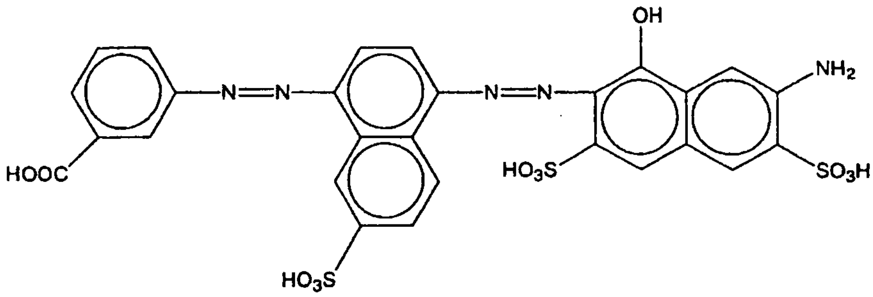

(3−3)

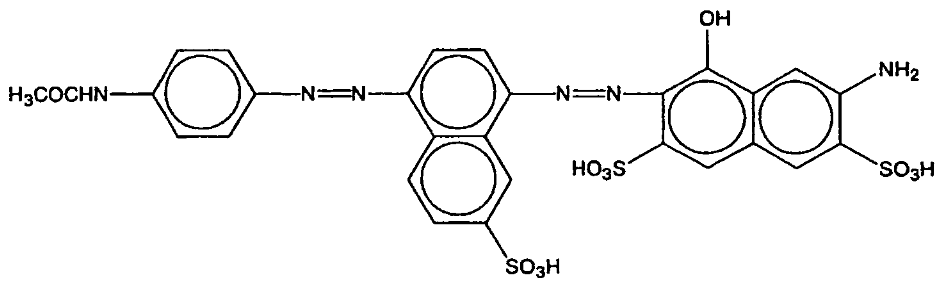

(3−4)

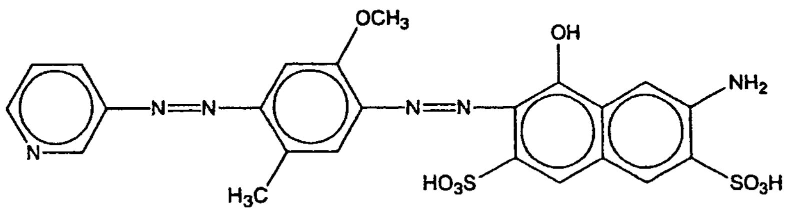

(3−5)

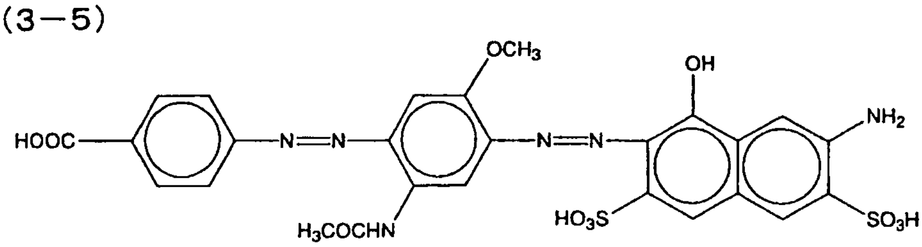

(3-6)

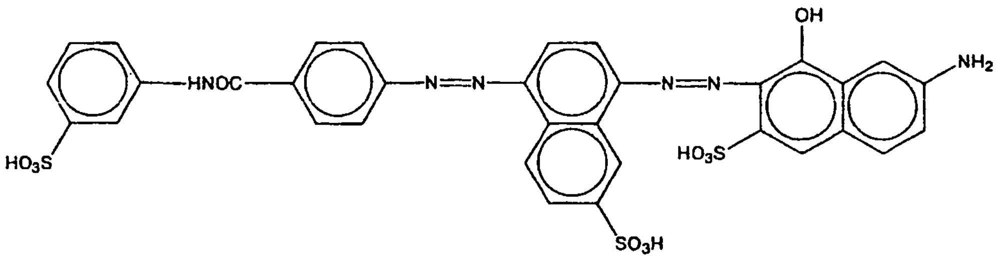

(3-7)

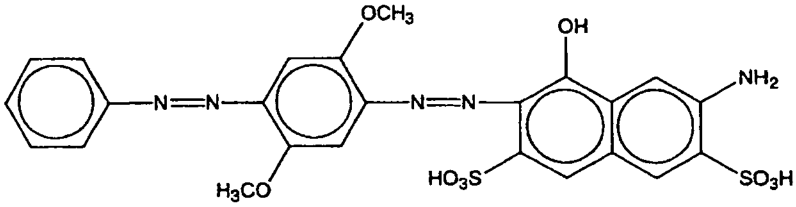

(3-8)

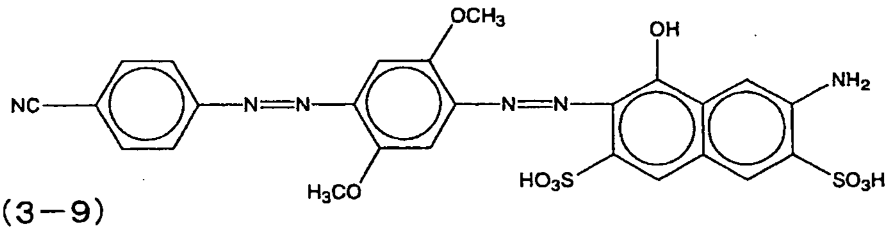

(3-9)

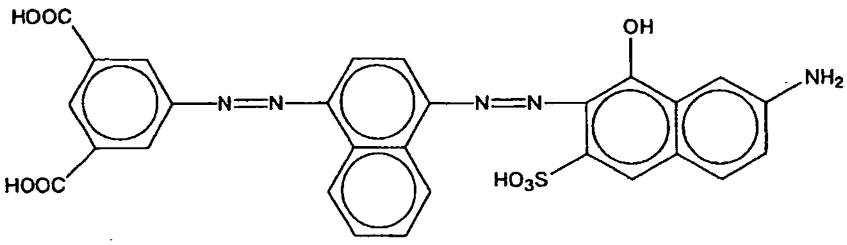

(3-10)

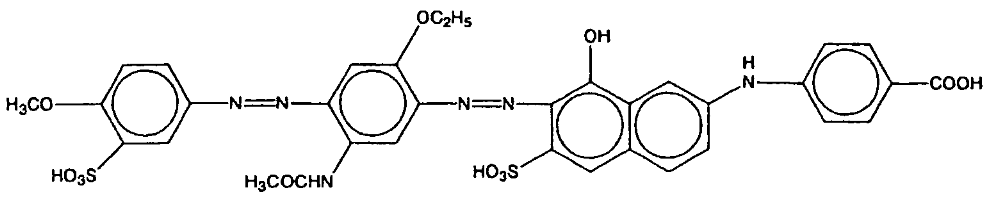

(3-11)

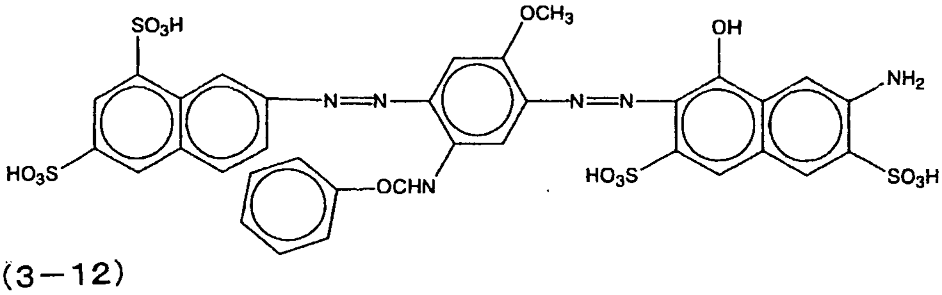

(3-12)

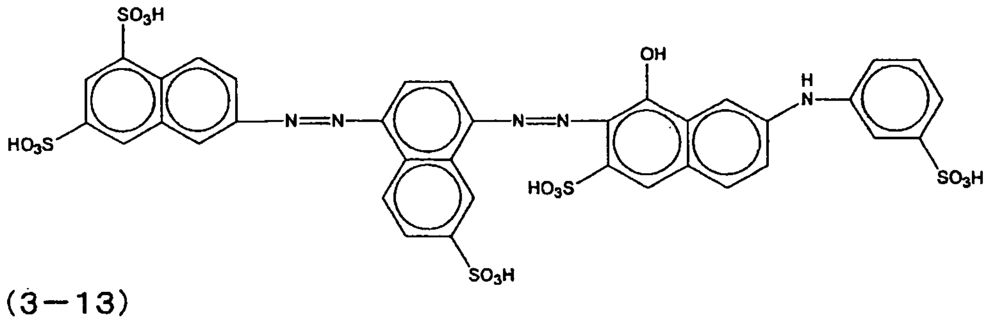

(3-13)

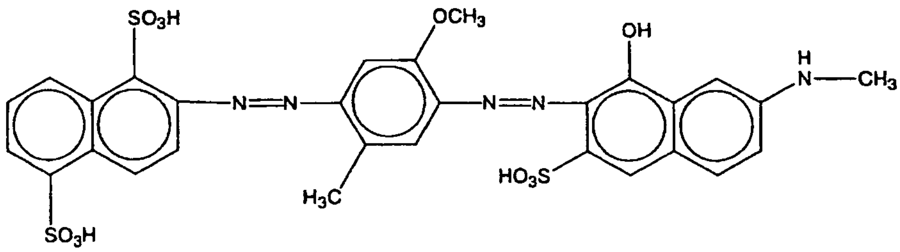

(3-14)

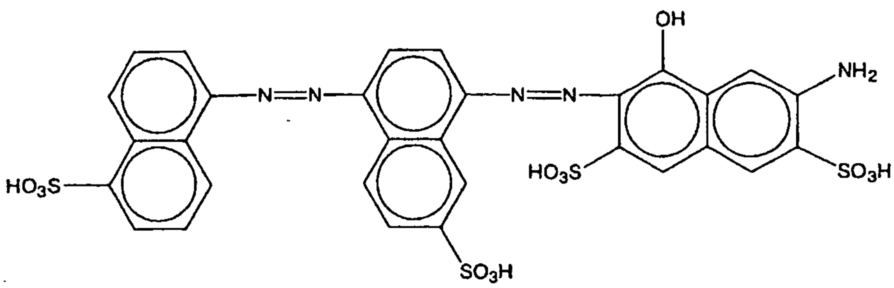

(3-15)

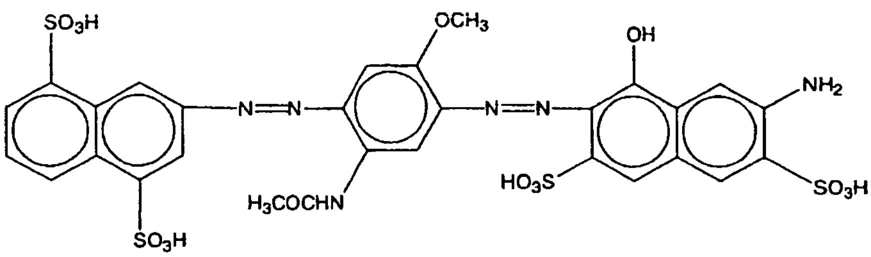

(3-16)

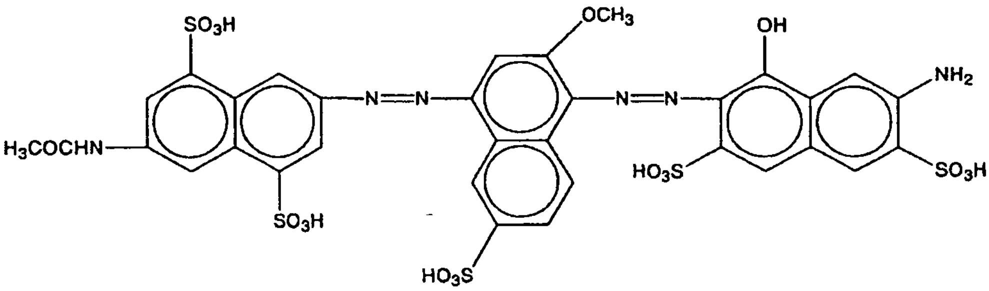

(3-17)

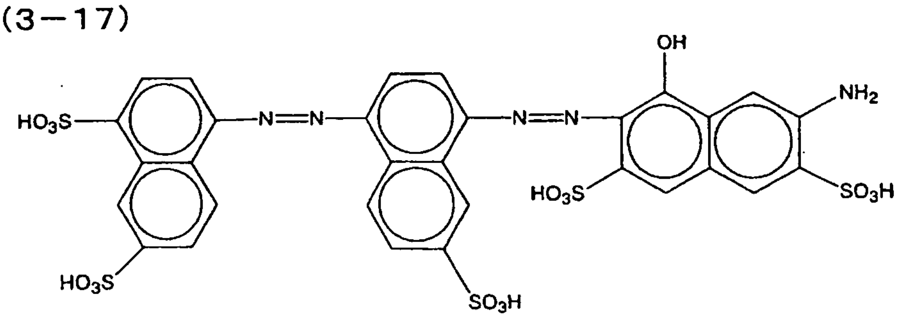

(3-18)

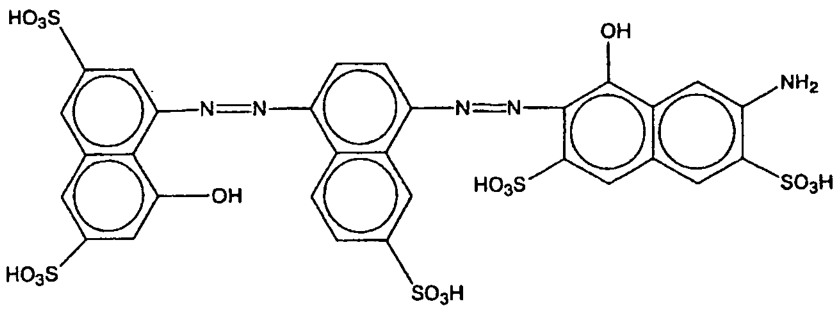

(3-19)

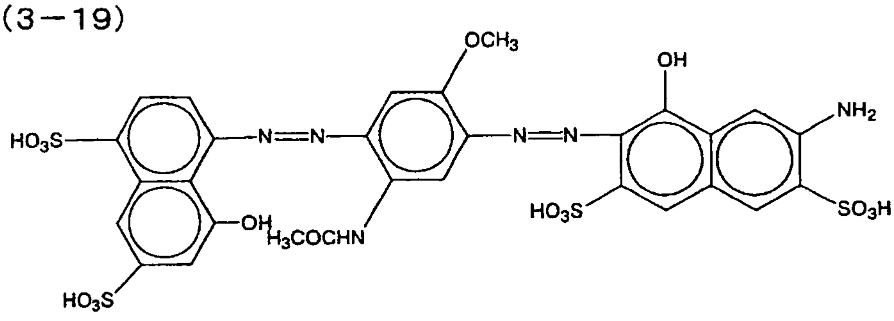

(3−20)

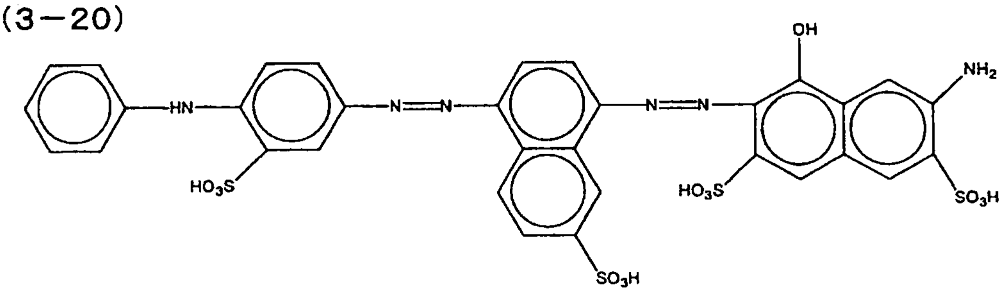

(3−21)

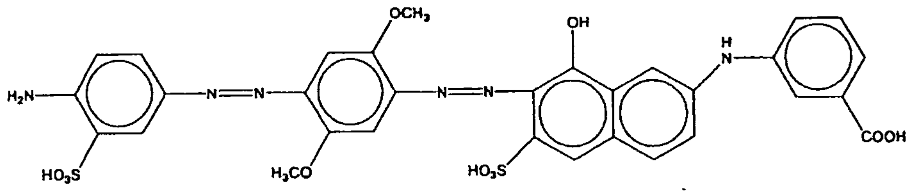

(3−22)

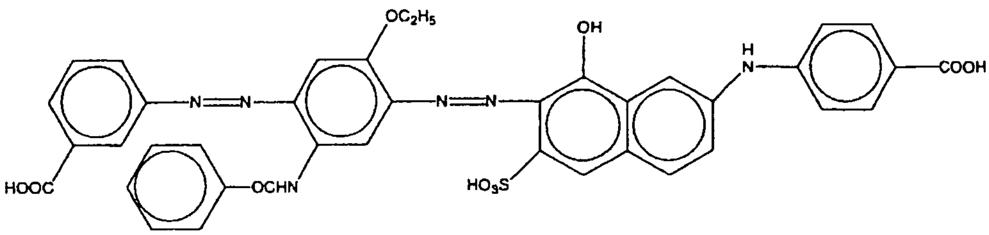

(3−23)

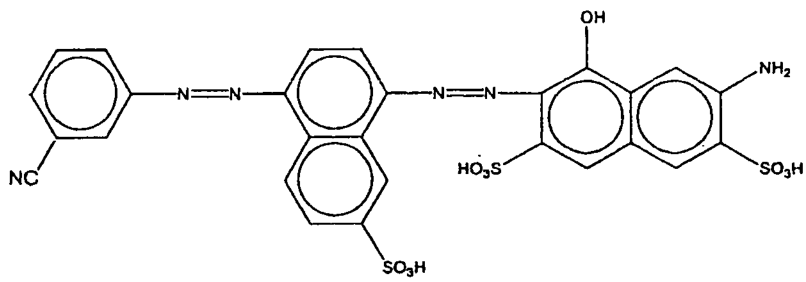

(3−24)

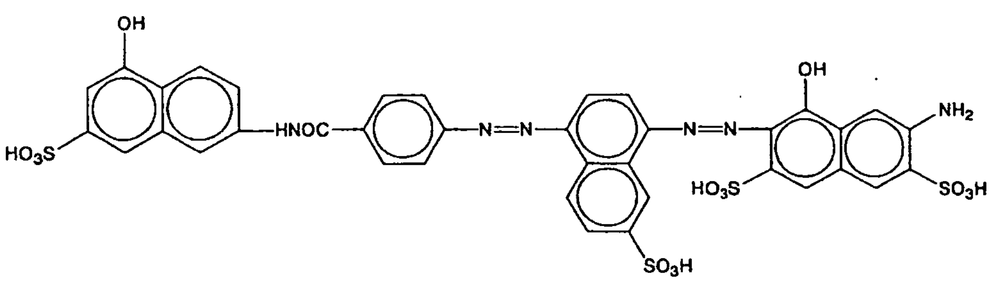

(3−25)

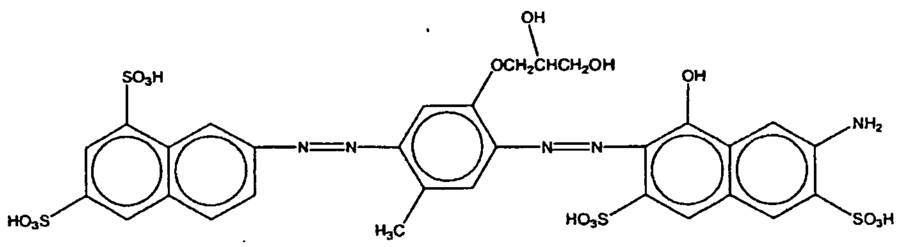

(3−26)

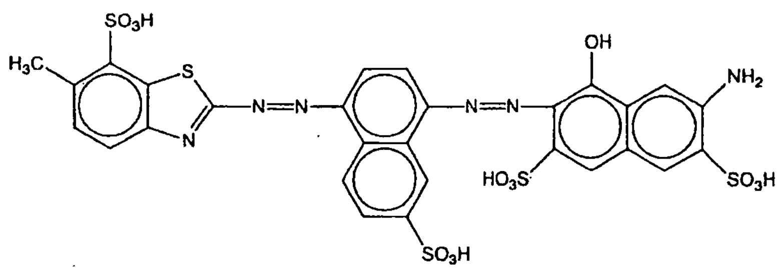

(3−27)

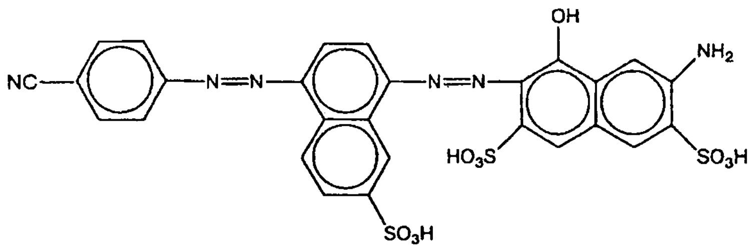

(3−28)

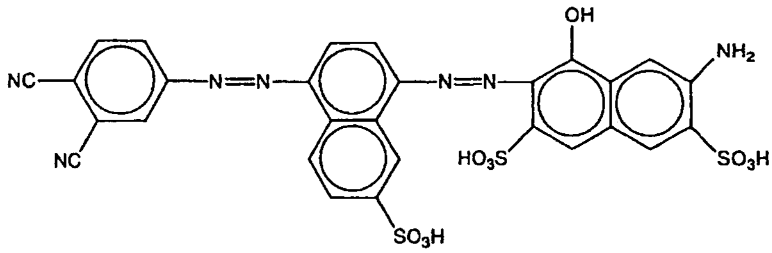

(3-29)

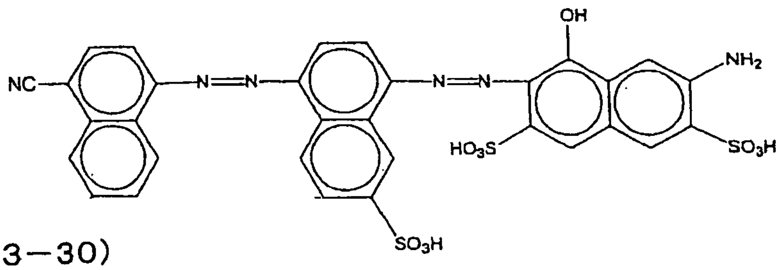

(3-30)

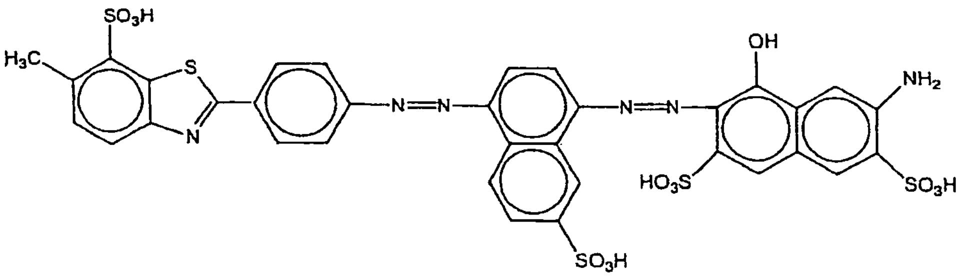

(3-31)

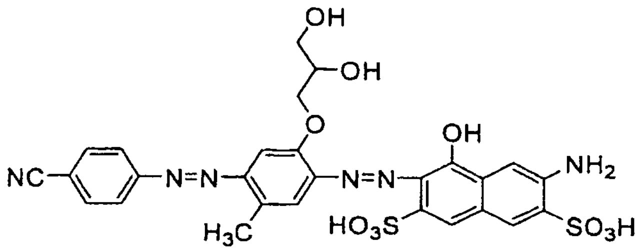

(3-32)

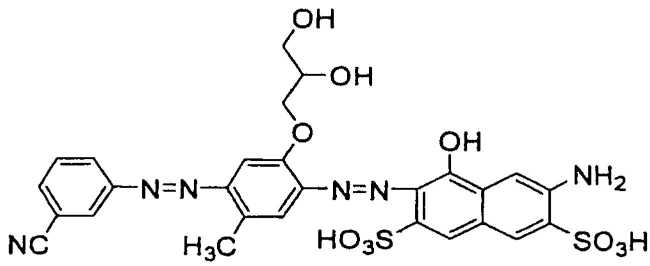

(3−33)

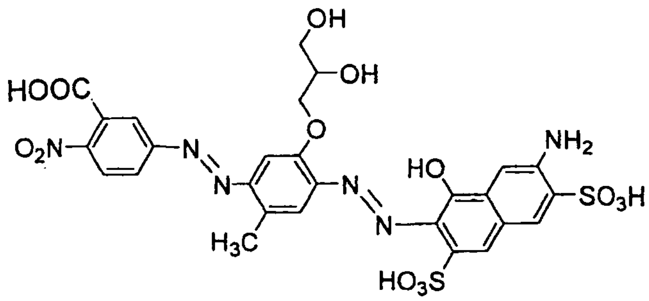

(3−35)

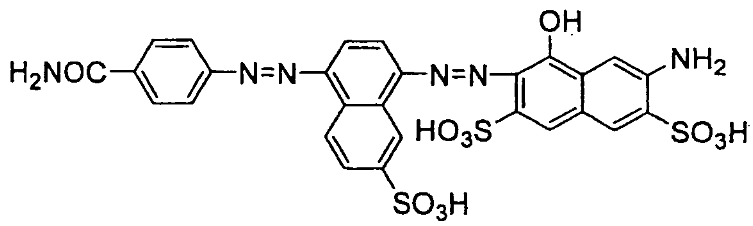

(3−36)

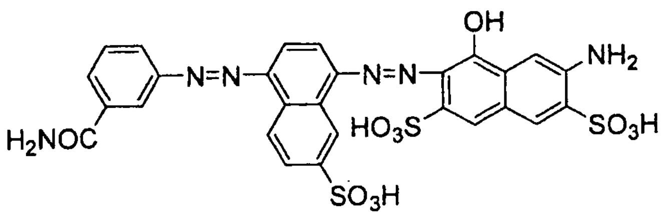

(3−37)

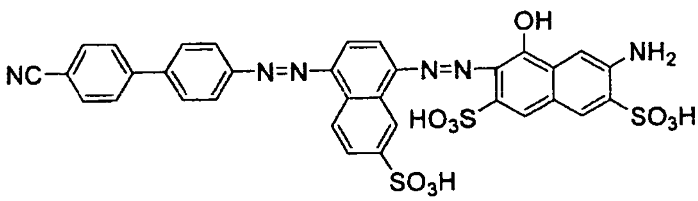

(3−38)

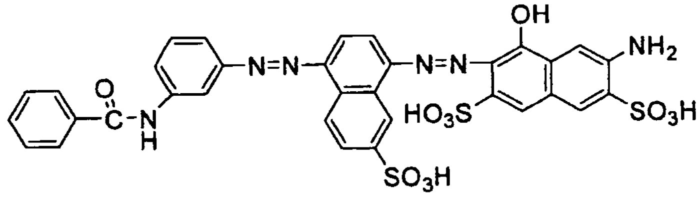

(3-39)

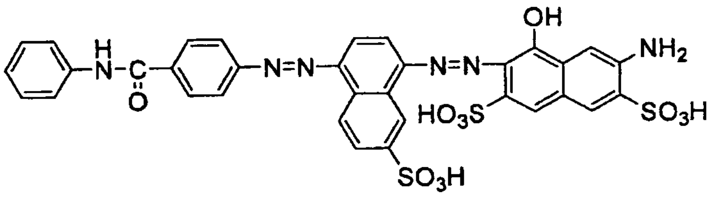

(3-40)

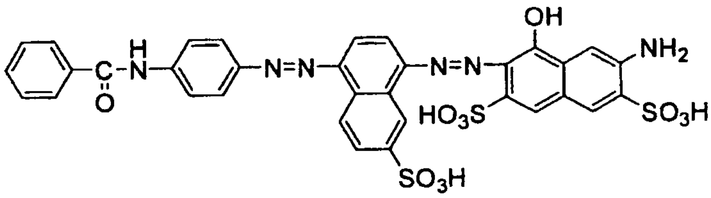

(3-41)

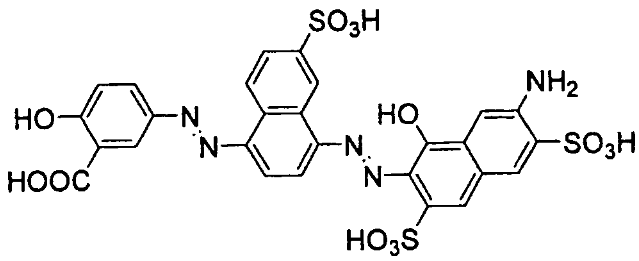

(3-42)

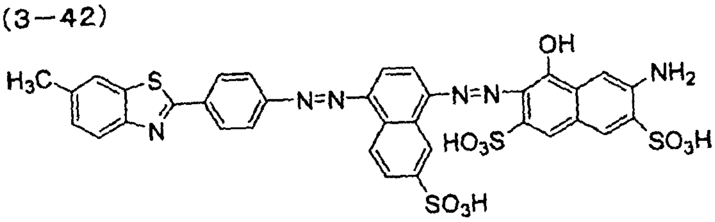

(3-43)

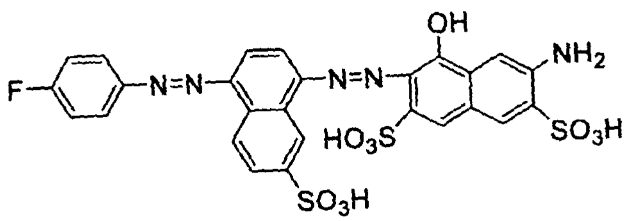

(3−44)

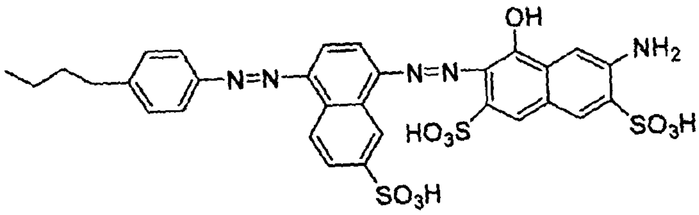

(3−45)

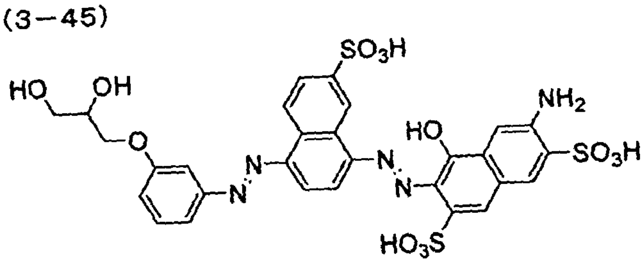

(3−46)

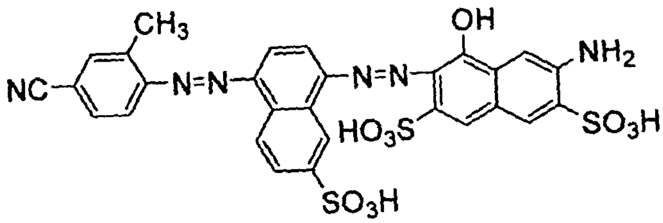

(3−47)

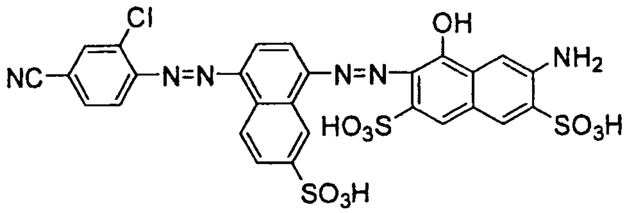

(3−48)

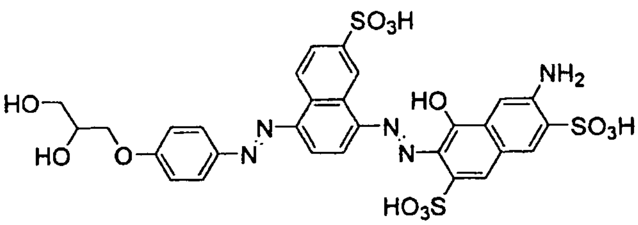

(3−49)

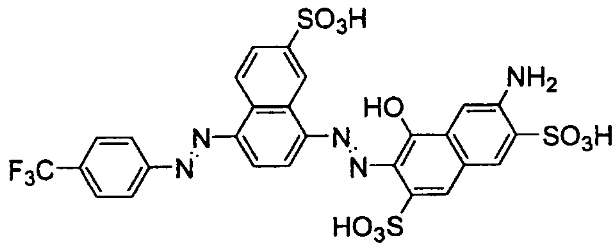

(3−50)

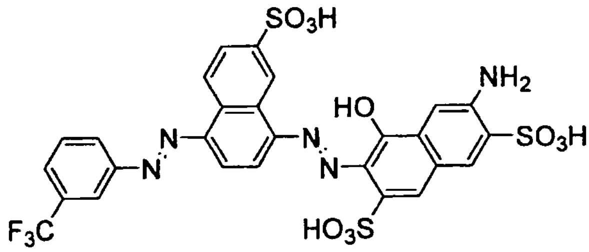

(3−51)

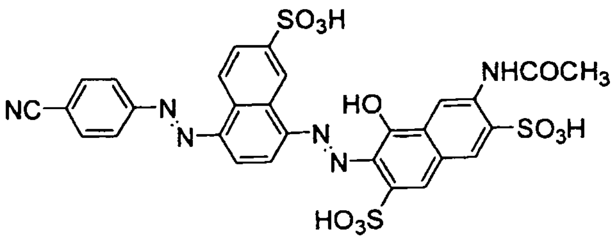

(3−52)

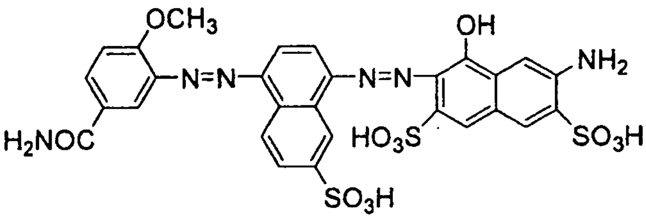

(3−53)

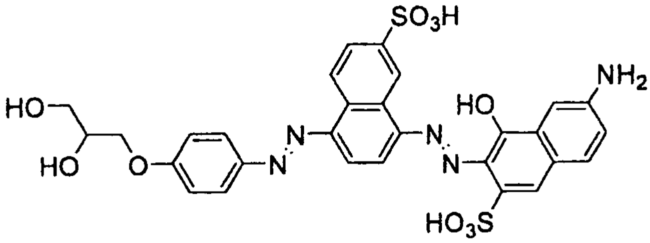

(3−54)

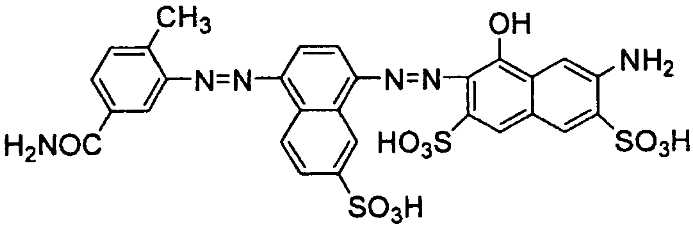

(3−55)

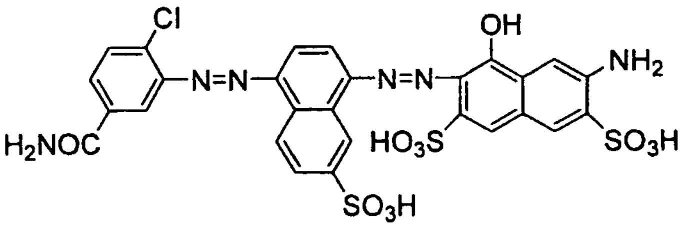

(3−56)

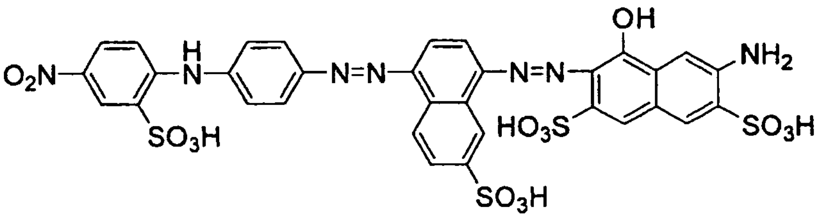

(3−57)

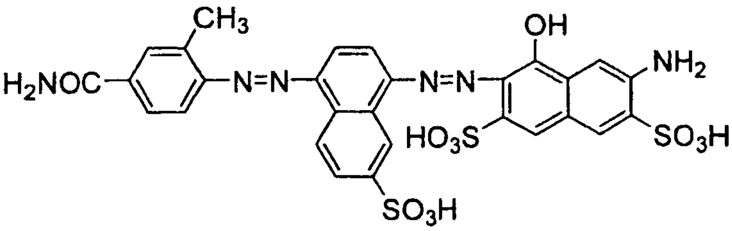

(3−58)

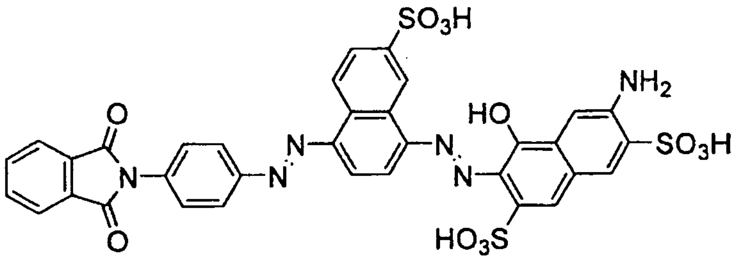

(3−59)

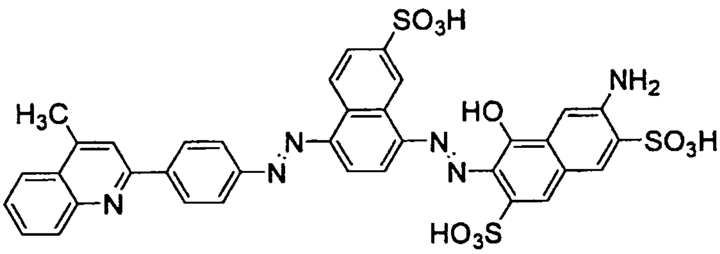

The azo dye represented by the above formula (3) may be produced in accordance with a known method. For example, dye No. (3-1) may be produced in accordance with the following steps (a3) and (b3).

(a3) In accordance with a conventional method (for example, "Shin Senryo Kagaku (New Dye Chemical)", Yutaka Hosoda (published on December 21, 1973, GIHODO SHUPPAN Co., Ltd.), pages 396-409), a monoazo compound is produced from 3-aminobenzenesulfonic acid (metanillic acid) and 8-amino-naphthalenesulfonic acid (1,7-Cleves acid) by means of diazotization and coupling.

(b3) The obtained monoazo compound is subjected to diazotization and coupling reaction with 7-amino-1-naphthol-3,6-disulfonic acid (RR acid) in accordance with a conventional method in the same manner, and salting out is carried out with sodium chloride to obtain a desired dye No. (3-1). The obtained dye may be purified as the case requires.

As another specific example of the azo dye which is a dye for an anisotropic dye film, of which the free acid form is represented by the above formula (I) of the present invention, a water soluble black dichroic azo dye represented by the following formula (4) may be mentioned:

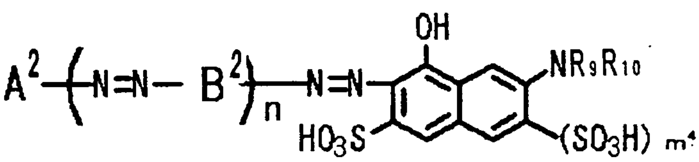

(4)

wherein $A^2$ is any of groups of the following formulae (4-a), (4-b) and (4-c), which may have a substituent:

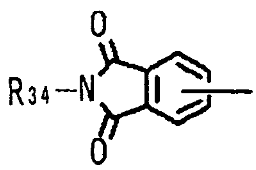

(4−a)

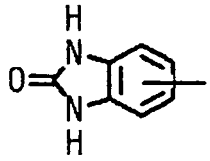

(4 − b)

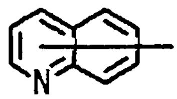

(4 − c)

(wherein $R_{34}$ is a hydrogen atom, an alkyl group which may have a substituent, or a phenyl group which may have a substituent),
$B^2$ is a bivalent aromatic hydrocarbon group which may have a substituent, or a bivalent aromatic heterocyclic group containing a nitrogen atom as the hetero atom; each of $R_9$ and $R_{10}$ which are independent of each other, is a hydrogen atom, an alkyl group which may have a substituent, a phenyl group which may have a substituent, or an acyl group which may have a substituent; $m^4$ is 0 or 1, and n is 1 or 2;
provided that when n is 2, a plurality of $B^2$ in one molecule may be the same or different.

The azo dye, as evident from $A^2$ in the formula (4), has a special structure having e.g. hydrogen bond-forming properties at the molecular terminal, and has such characteristics that the respective molecules are likely to be in an association state. It is considered that a highly lyotropic liquid crystalline state can be formed by such characteristics.

Further, many of the azo dyes are black, and a composition containing such a dye can provide a high degree of molecular orientation state by means of a process specific to the wet system film-forming method i.e. a lamination process by e.g. coating on the surface of a substrate. This means that it is possible to form an achromatic dye film with high anisotropy.

In the case of a conventional wet system film-forming method, an achromatic anisotropic dye film is obtained by combination of plural dyes in many cases. However, the azo dye of the present invention has a specific dye structure as described above, and thus forms a highly lyotropic liquid crystalline state and provides a high degree of molecular orientation state, and it is usually possible to provide black color only with one dye. Accordingly, an anisotropic dye film containing the azo dye can function as an anisotropic dye film having high dichroism.

Now, the azo dye represented by the above formula (4) of the present invention will be described below.

($A^2$)

In the formula (4), $A^2$ is any of groups of the above formulae (4-a), (4-b) and (4-c). In the formula, $R_{34}$ is a hydrogen atom, an alkyl group which may have a substituent, or a phenyl group which may have a substituent. Specifically, the alkyl group (preferably a $C_{1-4}$ alkyl group) which may have a substituent may, for example, be a methyl group, an ethyl group, a n-propyl group or a 1-hydroxyethyl group. The substituent of the alkyl group or the phenyl group may, for example, be an alkyl group such as a methyl group or an ethyl group, an alkoxy group such as a methoxy group or an ethoxy group, a hydroxy group, a nitro group, a sulfo group, a carboxy group, a halogen atom, an amino group such as an amino group or a methylamino group, an amide group or a cyano group.

Further, each of the groups represented by (4-a), (4-b) and (4-c) may further have a substituent, and the substituent may, for example, be a hydroxy group, a sulfo group, a carboxy group or a methyl group. The group is preferably non-substituted or substituted by a sulfo group, most preferably it is non-substituted.

$A^2$ is particularly preferably any of the following groups (4-a1), (4-b1), (4-c1) and (4-c2), with a view to obtaining more favorable dichroism:

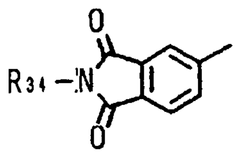

(4 − a 1)

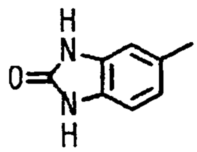

(4-b1)

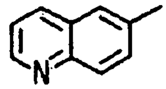

(4-c1)

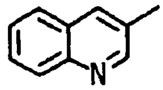

(4-c2)

In the formula, $R_{34}$ is as defined for the formula (4). Each of the groups (4-a1), (4-b1), (4-c1) and (4-c2) may further have a substituent in the same manner as (4-a), (4-b) and (4-c), and the substituent may, for example, be a hydroxy group, a sulfo group, a carboxy group or a methyl group. Preferably the group is non-substituted or substituted by a sulfo group, most preferably it is non-substituted.

($B^2$)

$B^2$ is a bivalent aromatic hydrocarbon group which may have a substituent or a bivalent aromatic heterocyclic group containing as the hetero atom a nitrogen atom. In a case where $m^4$ is 2, a plurality of $B^2$ in one molecule may be the same or different.

Specifically, the aromatic hydrocarbon group is preferably a bivalent group such as a phenylene group or a naphthylene group.

Further, specifically, the bivalent aromatic heterocyclic group containing as the hetero atom a nitrogen atom is preferably a bivalent group such as a quinolinediyl group or an isoquinolinediyl group. Particularly preferred is a phenylene group, a naphthylene group or a quinolinediyl group.

The phenylene group is preferably a 1,4-phenylene group, the naphthylene group is preferably a 1,4-naphthylene group, the quinolinediyl group is preferably a 5,8-quinolinediyl group, and the isoquinolinediyl group is preferably a 5,8-isoquinolinediyl group, with a view to obtaining the interaction of dye molecules.

The substituent which the aromatic hydrocarbon group or the aromatic heterocyclic group may have may, for example, be an alkyl group (preferably a $C_{1-4}$ alkyl group (such as a methyl group, an ethyl group, a n-propyl group, a hydroxyethyl group or a 1,2-dihydroxypropyl group)) which may have a substituent, an alkoxy group (preferably a $C_{1-4}$ alkoxy group (such as a methoxy group, an ethoxy group, a n-propoxy group, a n-butoxy group, a hydroxyethoxy group or a 1,2-dihydroxypropoxy group)) which may have a substituent, an acylamino group (preferably a $C_{2-7}$ acylamino group (such as an acetylamino group or a benzoylamino group)) which may have a substituent, a hydroxy group or a sulfo group.

The substituent may further have a substituent, such as a group exemplified as the above substituents.

Particularly in a case where $B^2$ is a phenylene group, the substituent is preferably a hydrogen bond-forming group or a group having low polarity such as an alkyl group, an alkoxy group or an acylamino group, in view of improvement in association properties by the interaction in formation of lyotropic liquid crystals. Specific examples and preferred examples of the alkyl group, the alkoxy group and the acylamino group are the same as those exemplified as substituents of the aromatic hydrocarbon group and the aromatic heterocyclic group. Further, each of the alkyl group, the alkoxy group and the acylamino group may further have a substituent, and specifically, it may be groups exemplified as substituents of the aromatic hydrocarbon group and the aromatic heterocyclic group.

Further, in a case where $B^2$ is a naphthylene group, the substituent is preferably a hydroxy group, a sulfo group or an alkoxy group, in view of improvement in association properties by the interaction in formation of lyotropic liquid crystals. Specific examples and preferred examples of the alkoxy group are the same as those exemplified as substituents of the aromatic hydrocarbon group and the aromatic heterocyclic group. Further, the alkoxy group may further have a

substituent, and specifically, it may be groups exemplified as substituents of the aromatic hydrocarbon group and the aromatic heterocyclic group, and it is particularly preferably a hydroxy group or an alkoxy group.

Further, in a case where $B^2$ is a quinolinediyl group or an isoquinolinediyl group, the substituent is the same as those of the naphthylene group, and particularly preferably, $B^2$ is non-substituted or substituted by a carboxy group.

($R_9$ and $R_{10}$)

In the formula (4), each of $R_9$ and $R_{10}$ which are independent of each other, is a hydrogen atom, an alkyl group (preferably a $C_{1-4}$ alkyl group (such as a methyl group or an ethyl group)) which may have a substituent, a phenyl group which may have a substituent, or an acyl group (such as an acetyl group or a benzoyl group) which may have a substituent.

Particularly preferred is e.g. an amino group wherein $R_9$ and $R_{10}$ are hydrogen atoms, an alkylamino group wherein $R_9$ is a hydrogen atom and $R_{10}$ is an alkyl group, or an arylamino group wherein $R_9$ is a hydrogen atom and $R_{10}$ is a phenyl group. Particularly preferably, both $R_9$ and $R_{10}$ are hydrogen atoms.

The substituent which the alkyl group, the phenyl group or the acyl group may have may be a hydroxy group, a carboxy group, or a sulfo group.

(n and $m^4$)

$m^4$ is 0 or 1, and n is 1 or 2. The azo dye of the present invention is usually a disazo dye or a trisazo dye.

(Formula (4-A))

The dye represented by the formula (4) of the present invention is particularly preferably a dye represented by the following formula (4-A).

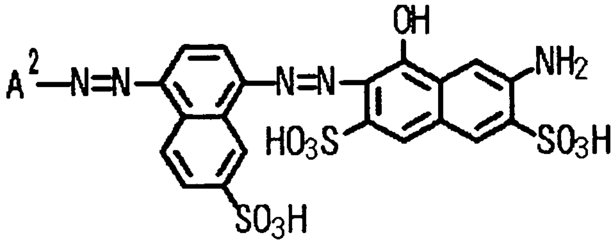

(4-A)

wherein $A^2$ is as defined for the formula (4).

(Molecular weight)

The molecular weight of the dye represented by the formula (4) is preferably at least 650, and preferably at most 1,500, more preferably at most 1,100, in the free acid form.

In the dye represented by the formula (4), in the dye structure, a structure having e.g. hydrogen bond-forming properties at the molecular terminal is specified, whereby association properties are improved as described above, and a highly lyotropic liquid crystalline state can be formed. Accordingly, the dye represented by the formula (4) of the present invention is suitable as a dye for an anisotropic dye film to be formed by a wet system film-forming method. Further, since it has a high dichroic ratio, by using a composition containing the dye, an anisotropic dye film having high dichroism can be obtained.

The dye represented by the formula (4) is usually a water soluble dye.

The present invention provides an azo dye, of which the free acid form is represented by the following formula (6):

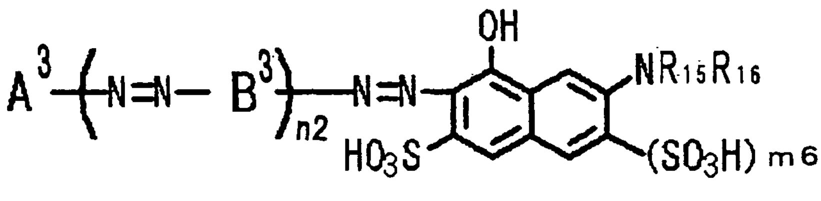

(6)

wherein $A^3$ is any of groups of the following formulae (6-a), (6-b) and (6-c), which may have a substituent:

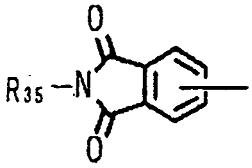

(6 − a)

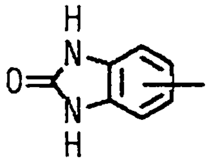

(6 − b)

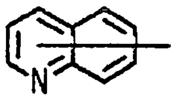

(6 − c)

(wherein $R_{35}$ is a hydrogen atom, an alkyl group which may have a substituent, or a phenyl group which may have a substituent),

$B^3$ is a bivalent aromatic hydrocarbon group which may have a substituent, or a bivalent aromatic heterocyclic group containing as the hetero atom a nitrogen atom; each of $R_{15}$ and $R_{16}$ which are independent of each other, is a hydrogen atom, an alkyl group which may have a substituent, a phenyl group which may have a substituent, or an acyl group which may have a substituent; $m^6$ is 0 or 1, and n2 is 1 or 2;
provided that when n2 is 2, a plurality of $B^3$ in one molecule may be the same or different.

In the above formula (6), $A^3$ is more preferably any of groups of the following formulae (6a-1), (6-b1), (6-c1) and (6-c2), which may have a substituent:

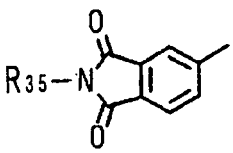

(6 − a 1)

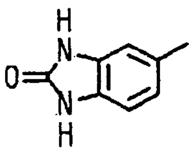

(6 − b 1)

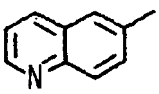

(6 − c 1)

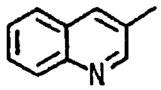

(6 − c 2)

wherein $R_{35}$ is as defined for the formula (6).

In the formula (6), $A^3$, $B^3$, $R_{15}$ and $R_{16}$ are as defined for $A^2$, $B^2$, $R_9$ and $R_{10}$ in the above formula (4), respectively. Further, $R_{35}$ is as defined for $R_{34}$ in the above formula (4) .

Specific examples of the dyes represented by the above formulae (4) and (6) of the present invention include dyes having structures represented by the following formulae (4-1) to (4-23), (4-26) and (4-29) to (4-33) in the free acid form. However, the dye is not limited thereto. In (4-15), $C_3H_7$-n represents a n-propyl group:

(4−1)

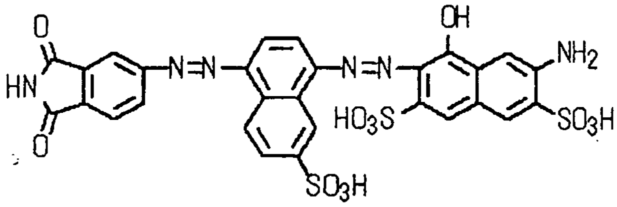

(4−2)

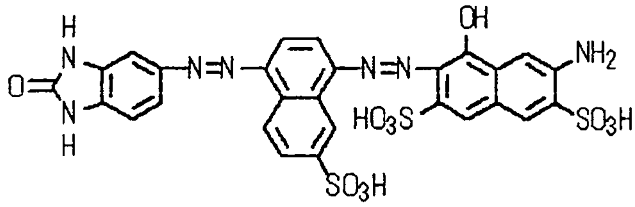

(4−3)

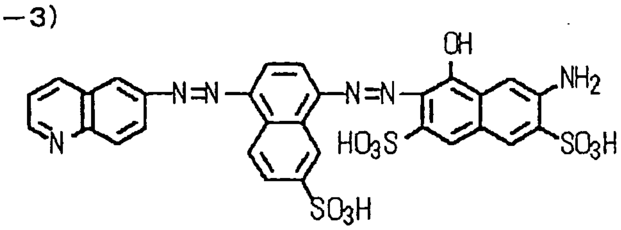

(4−4)

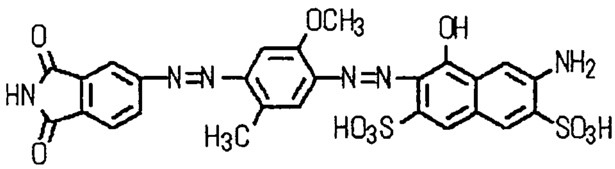

(4-5)

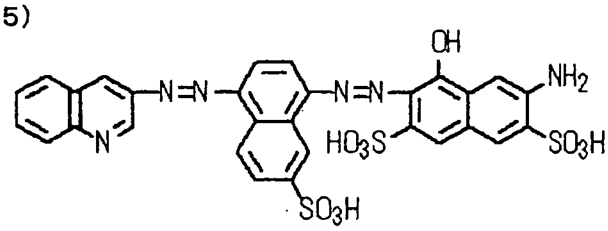

(4-6)

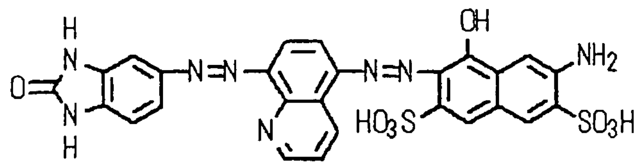

(4-7)

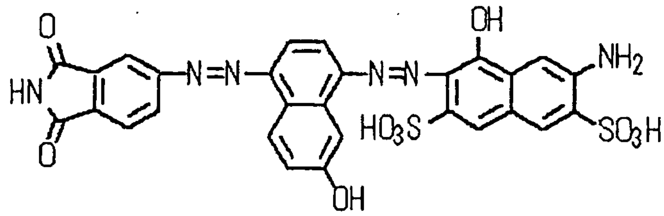

(4-8)

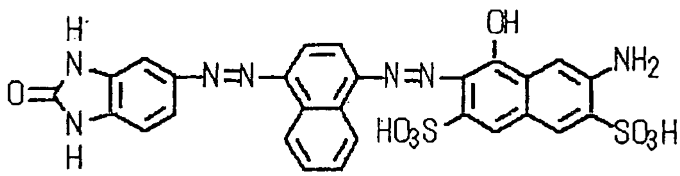

(4-9)

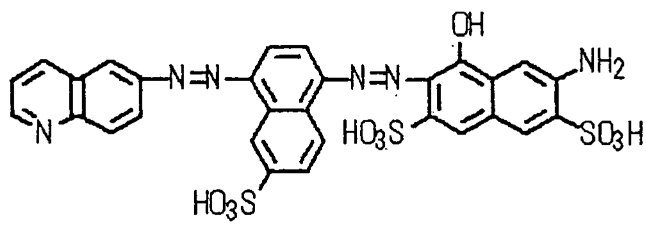

(4−10)

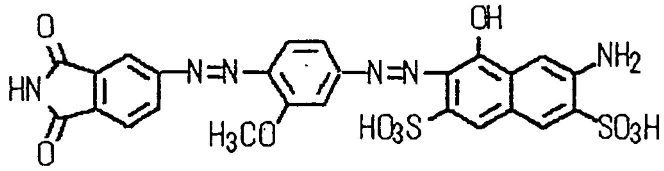

(4−11)

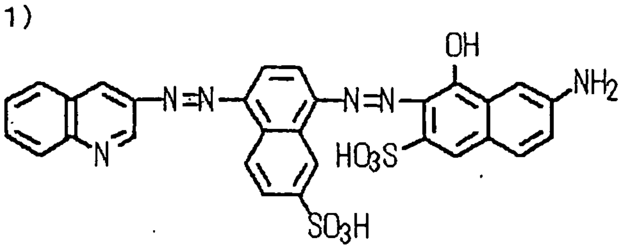

(4−12)

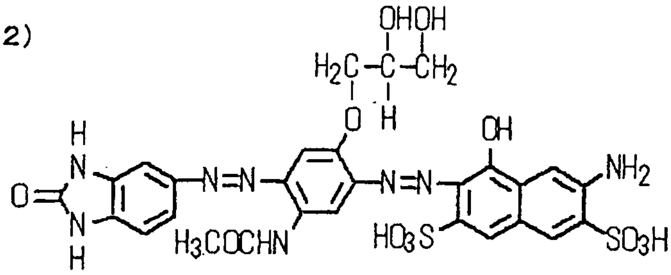

(4−13)

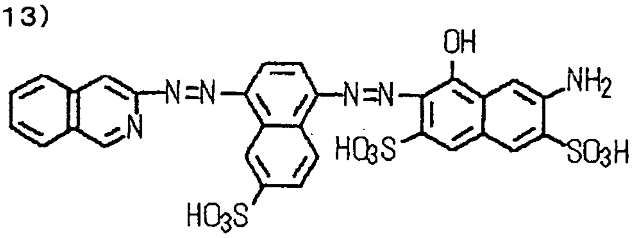

(4−14)

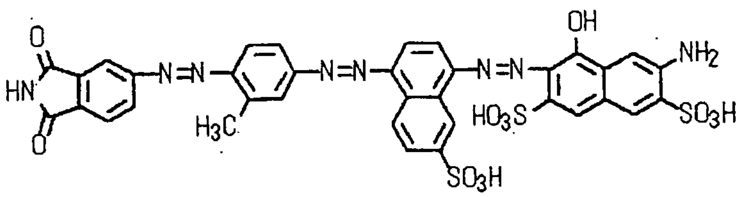

(4－15)

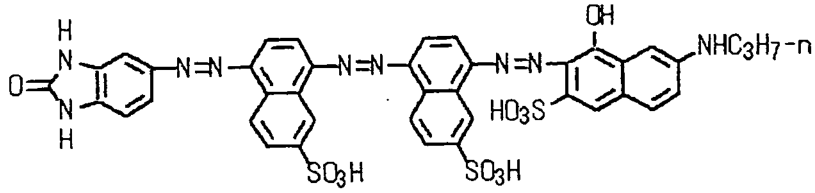

(4－16)

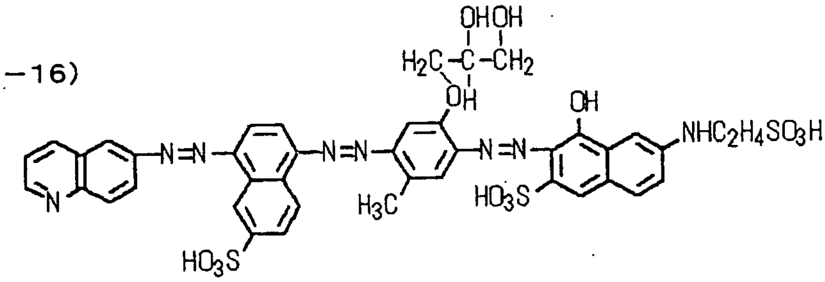

(4－17)

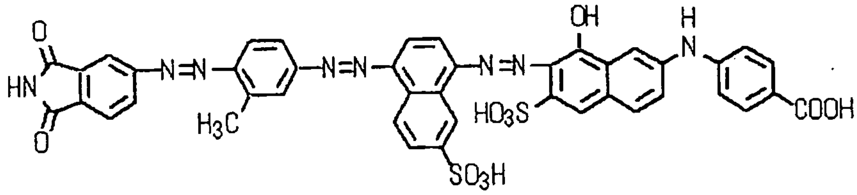

(4－18)

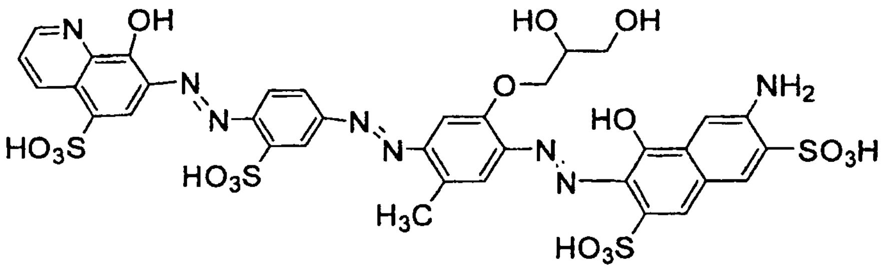

(4－19)

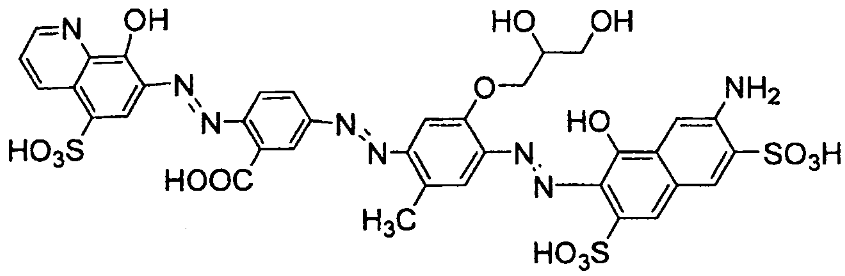

(4-20)

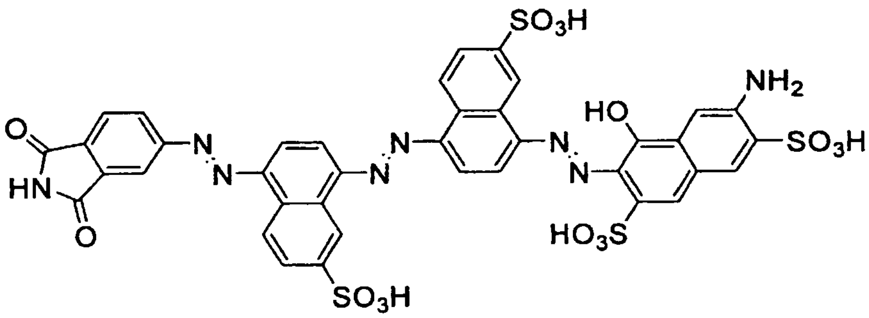

(4-21)

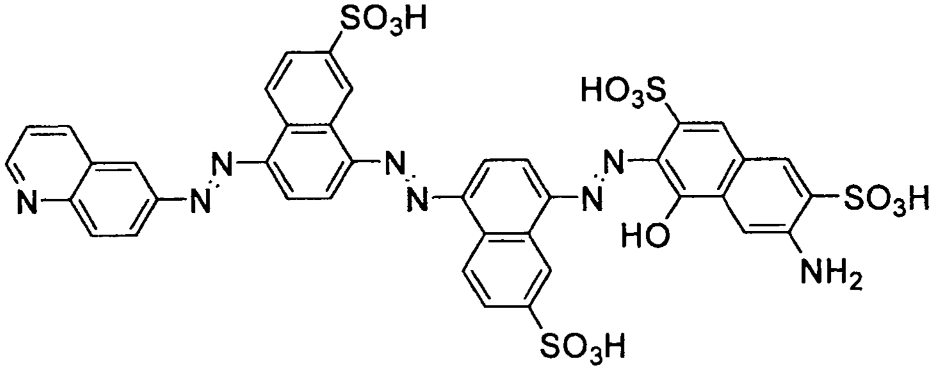

(4-22)

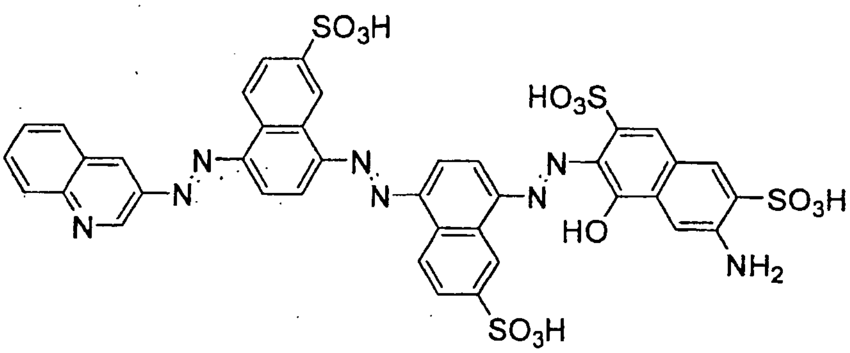

(4-23)

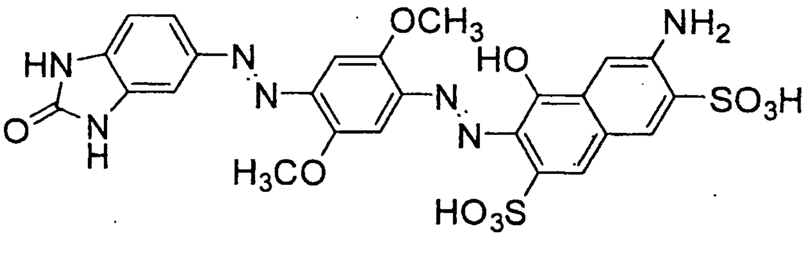

(4−26)

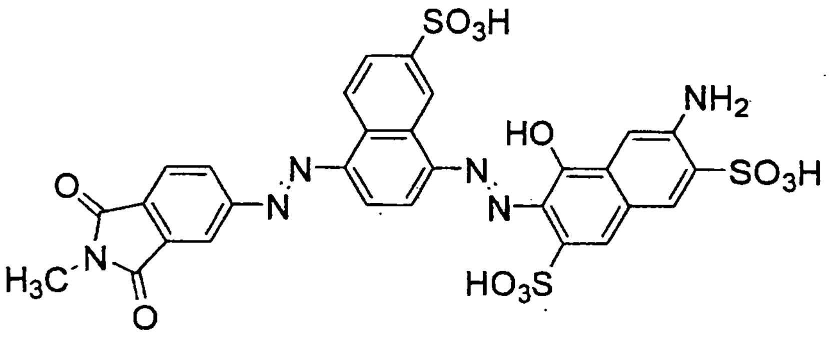

(4−29)

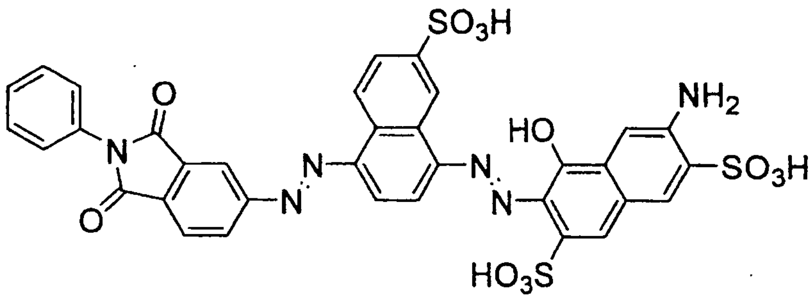

(4−30)

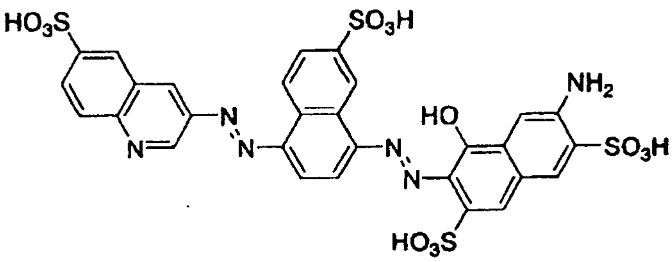

(4−31)

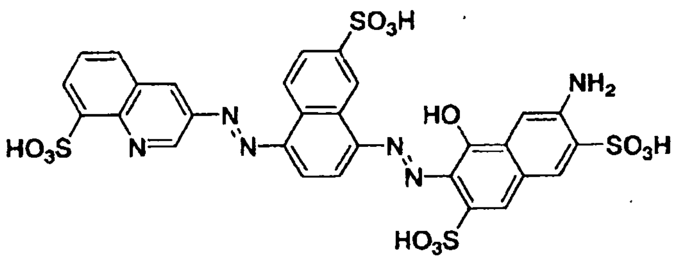

(4-32)

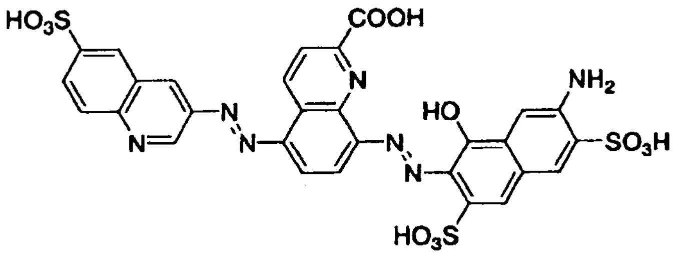

(4-33)

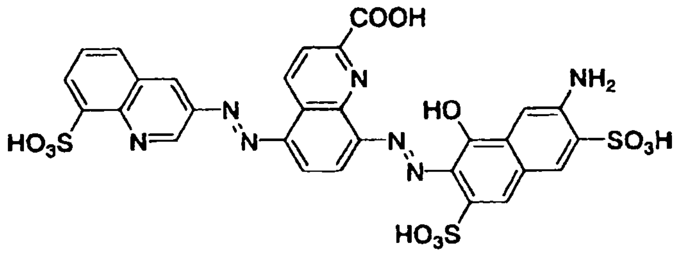

The azo dye represented by the formula (4) may be produced in accordance with a known method. For example, dye No. (4-1) may be produced in accordance with the following steps (a4) and (b4).

(a4) In accordance with a conventional method (for example, "Shin Senryo Kagaku (New Dye Chemical)", Yutaka Hosoda (published on December 21, 1973, GIHODO SHUPPAN Co., Ltd.), pages 396-409), a monoazo compound is produced from 4-aminophthalimide and 8-amino-2-naphthalenesulfonic acid (1,7-Cleves acid) by means of diazotization and coupling.

(b4) The obtained monoazo compound is subjected to diazotization and coupling reaction with 7-amino-1-naphthol-3,6-disulfonic acid (RR acid) in accordance with a conventional method in the same manner, and salting out is carried out with sodium chloride to obtain a desired dye No. (4-1).

Particularly, the dye represented by the above formula (4-A) of the present invention forms lyotropic liquid crystals in the aqueous solution, and accordingly an anisotropic dye film having high dichroism can be prepared with it, and it is a useful dye particularly suitable for the wet system film-forming method.

(Excitation purity)

The dyes of the present invention represented by the above formula (I), specifically the formulae (1) to (5) are dyes for an anisotropic dye film to be formed by a wet system film-forming method, and preferably provide black color, and particularly preferably they are dyes for an anisotropic dye film having an excitation purity of from 0 to 18%, preferably from 0 to 12%. Namely, by employing a dye having an excitation purity of from 0 to 18%, disturbance of the molecular orientation by mixing of different molecules will not occur, and high dichroism can be obtained. In the present invention, preferred is a dye having an excitation purity of at least 0% and at most 18%, and the excitation purity is more preferably at most 12%, furthermore preferably at most 9%, most preferably at most 6%.

Further, with respect to the anisotropic dye film also, the excitation purity is preferably at least 0% and at most 18%, more preferably at most 12%, furthermore preferably at most 9%, most preferably at most 6%.

Here, the excitation purity is obtained in such a manner that the chromaticity coordinates N of standard illuminant and the chromaticity coordinates C of the obtained dye in the chromaticity diagram are connected with a straight line, and the wavelength corresponding to the intersection point of the extension of the straight line and the spectrum locus is taken as the dominant wavelength, and the excitation purity is calculated from the proportion at the respective points. The chromaticity coordinate C can be obtained in such a manner that the dye is added to water to obtain a dye aqueous solution, the visible light transmittance of the aqueous solution is measured by a spectrophotometer, and the chromaticities x and y in the CIE 1964 supplementary standard colorimetric system under standard illuminant $D_{65}$ are calculated.

The excitation purity of the dye in the present invention means one measured and calculated as a dye aqueous

solution obtained by adding the dye to water, and the excitation purity of the anisotropic dye film means one measured and calculated as a film formed by applying the composition for an anisotropic dye film to a substrate.

Further, the calculation method may be in accordance with a known method as disclosed in e.g. "New Color Science Handbook" edited by THE COLOR SCIENCE ASSOCIATION OF JAPAN (UNIVERSITY OF TOKYO PRESS, published on November 25, 1989 (second edition)), pages 104 to 105.

The dyes to be used in the present invention may be used as the free acid forms represented by the above formula (I), specifically the formulae (1) to (4) and (5), or part of the acidic groups may be in a salt form. Otherwise, a dye in a salt form and a dye in a free acid form may coexist. Further, when the dye is obtained in a salt form at the time of the production, it may be used as it is, or it may be converted into a desired salt form. As the method of converting the salt form, a known method may optionally be employed, and the following methods may, for example, be mentioned.

1) A method of adding a strong acid such as hydrochloric acid to an aqueous solution of the dye obtained in a salt form, to precipitate the dye in a free acid form, and then neutralizing the dye acidic groups with an alkali solution having desired counter ions (such as lithium hydroxide aqueous solution) to carry out salt exchange.
2) A method of adding a normal salt having desired counter ions (such as lithium chloride) in a large excess to an aqueous solution of the dye obtained in a salt form, to carry out salt exchange in the form of a salted out cake.
3) A method of treating an aqueous solution of the dye obtained in a salt form with a strongly acidic cation exchange resin to precipitate the dye in a free acid form, and neutralizing the dye acidic groups with an alkali solution having desired counter ions (such as lithium hydroxide aqueous solution) to carry out salt exchange.
4) A method of reacting an aqueous solution of the dye obtained in a salt form with a strongly acidic cation exchange resin which is preliminarily treated with an alkali solution having desired counter ions (such as lithium hydroxide aqueous solution) to carry out salt exchange.

With respect to the dye used in the present invention, whether the acidic group is in a free acid form or in a salt form depends on the pKa of the dye and the pH of the dye aqueous solution.

As examples of the above salt form, a salt of an alkali metal such as Na, Li or K, a salt of ammonium which may be substituted by an alkyl group or a hydroxyalkyl group, and a salt of an organic amine may be mentioned.

As examples of the organic amine, a $C_{1-6}$ lower alkylamine, a $C_{1-6}$ lower alkylamine substituted by a hydroxy group, and a $C_{1-6}$ lower alkylamine substituted by a carboxy group may, for example, be mentioned. In the case of such a salt form, the type is not limited to one type, and a plural types may be present.

The composition for an anisotropic dye film of the present invention contains the dye represented by the formula (I), specifically the formulae (1) to (4) and the formula (5) and a solvent. In the composition, the dye represented by any of the above formulae may be used alone, or dyes represented by any of the formulae may be used in combination, dyes represented by different formulae may be used in combination, or another dye may be blended to the extent of not decreasing the orientation, whereby anisotropic dye films having various hues can be produced.

Preferred examples of a dye to be blended include C.I. Direct Yellow 12, C.I. Direct Yellow 34, C.I. Direct Yellow 86, C.I. Direct Yellow 142, C.I. Direct Yellow 132, C.I. Acid Yellow 25, C.I. Direct Orange 39, C.I. Direct Orange 72, C.I. Direct Orange 79, C.I. Acid Orange 28, C.I. Direct Red 39, C.I. Direct Red 79, C.I. Direct Red 81, C.I. Direct Red 83, C.I. Direct Red 89, C.I. Acid Red 37, C.I. Direct Violet 9, C.I. Direct Violet 35, C.I. Direct Violet 48, C.I. Direct Violet 57, C.I. Direct Blue 1, C.I. Direct Blue 67, C.I. Direct Blue 83, C.I. Direct Blue 90. C.I. Direct Green 42, C.I. Direct Green 51 and C.I. Direct Green 59.

As the solvent to be used for the composition for an anisotropic dye film of the present invention, water, a water-miscible organic solvent or a mixture thereof is suitable. As specific examples of the organic solvent, an alcohol such as methyl alcohol, ethyl alcohol or isopropyl alcohol, a glycol such as ethylene glycol or diethylene glycol, or a cellosolve such as methyl cellosolve or ethyl cellosolve may, for example, be used alone or as a mixed solvent of at least two types thereof.

In a case where the dye is dissolved, the concentration is preferably at least 0.1 wt%, more preferably at least 0.5 wt%, and preferably at most 30 wt%, more preferably at most 25 wt%, particularly preferably at most 20 wt%, although it depends on the solubility of the dye and the formation concentration of the association state such as the lyotropic liquid crystalline state.

To the composition for an anisotropic dye film of the present invention, an additive such as a surfactant may be added as the case requires, in order to improve wettability and the coating properties on a substrate. As the surfactant, any of anionic, cationic and nonionic surfactants may be used. The addition concentration is usually preferably at least 0.05 wt% and at most 0.5 wt%.

The anisotropic dye film of the present invention is an anisotropic dye film formed by a wet system film-forming method, containing the dye of the present invention represented by the above formula (I), specifically the formulae (1) to (4) and the formula (5). Usually the anisotropic dye film of the present invention is obtained by formation on a substrate by a wet system film-forming method employing the composition for an anisotropic dye film of the present invention.

As described above, the azo dyes represented by the above formula (I), specifically the formulae (1) to (5) have a specific dye structure, and thereby form a highly lyotropic liquid crystalline state, provide a high degree of molecular orientation and show high dichroism. Therefore, the anisotropic dye film of the present invention is a useful dye film showing high dichroism.

The anisotropic dye film of the present invention has a high dichroic ratio, and the dichroic ratio is preferably at least 9, more preferably at least 12, particularly preferably at least 15.

The anisotropic dye film of the present invention may be an anisotropic dye film containing a dye, of which the free acid form is represented by the following formula (5), formed by a wet system film-forming method, and having a dichroic ratio of at least 40:

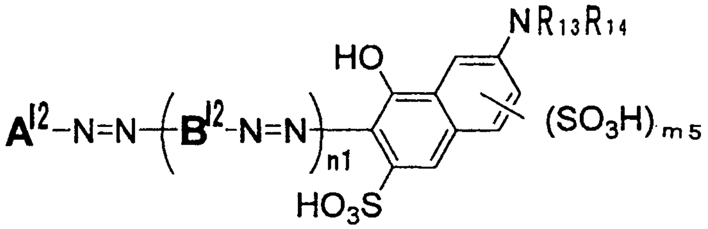

(5)

wherein $A^{12}$ is an aromatic hydrocarbon group which may have a substituent, or an aromatic heterocyclic group which may have a substituent; $B^{12}$ is a bivalent aromatic hydrocarbon group which may have a substituent, or a bivalent aromatic heterocyclic group which may have a substituent; each of $R_{13}$ and $R_{14}$ which are independent of each other, is a hydrogen atom, an alkyl group which may have a substituent, or a phenyl group which may have a substituent; $m^5$ is 0 or 1; and n1 is 1 or 2; provided that when n1 is 2, a plurality of $B^{12}$ in one molecule may be the same or different.

($A^{12}$)

In the formula (5), $A^{12}$ is an aromatic hydrocarbon group which may have a substituent or an aromatic heterocyclic group which may have a substituent. The aromatic hydrocarbon group may, for example, be a phenyl group or a naphthyl group, and the aromatic heterocyclic group may be an aromatic heterocyclic group containing as the hetero atom a nitrogen atom or a sulfur atom, such as a pyridyl group, a quinolyl group, a thiazolyl group, a benozothiazolyl group, a phthalimidoyl group or a quinolonyl group.

The substituent which such a group may have is preferably a hydrophilic group to be introduced to increase the solubility of the dye, or an electron-donating group or an electron-withdrawing group to be introduced to adjust the chromaticness. Specifically, it may, for example, be an alkyl group (preferably a $C_{1-4}$ alkyl group) which may be substituted, such as a methyl group, an ethyl group, a n-propyl group, a hydroxyethyl group or a 1,2-dihydroxypropyl group; or an alkoxy group (preferably a $C_{1-4}$ alkoxy group) which may be substituted, such as a methoxy group, an ethoxy group, a n-propoxy group, a n-butoxy group, a hydroxyethoxy group or a 1,2-dihydroxypropoxy group.

Further, it may, for example, be an amino group which may be substituted, such as an alkylamino group (preferably an amino group substituted by $C_{1-4}$ alkyl group) such as a methylamino group, an ethylamino group, a propylamino group or a dimethylamino group, a phenylamino group, or an acylamino group (preferably an amino group substituted by $C_{2-7}$ acyl group) such as an acetylamino group or a benzoylamino group; a substituted carbamoyl group such as a phenylaminocarbonyl group or a naphthylaminocarbonyl group; a carboxy group; a sulfo group; a hydroxy group; a cyano group; or a halogen atom. Among these substituents, preferred is a sulfo group, a hydroxy group, a carboxy group, a cyano group, a carbamoyl group, a methoxy group, a methyl group or a chlorine atom.

The above alkyl group, alkoxy group, phenyl group and naphthyl group may further have a substituent, and the substituent may, for example, be a hydroxy group, a sulfo group or an alkoxy group.

($B^{12}$)

$B^{12}$ is a bivalent aromatic hydrocarbon group which may have a substituent or a bivalent aromatic heterocyclic group which may have a substituent. The bivalent aromatic hydrocarbon group may, for example, be a phenylene group or a naphthylene group, and the bivalent aromatic heterocyclic group may be an aromatic heterocyclic group containing as the hetero atom a nitrogen atom, such as a quinolinediyl group or an isoquinolinediyl group. The substituent which these groups may have may be the same group as substituents which the group represented by $A^{12}$ may have.

($R_{13}$ and $R_{14}$)

Each of $R_{13}$ and $R_{14}$ which are independent of each other, is a hydrogen atom, an alkyl group (preferably a $C_{1-4}$ alkyl group (such as a methyl group or an ethyl group)) which may have a substituent, a phenyl group which may have a substituent, or an acyl group (such as an acetyl group or a benzoyl group) which may have a substituent.

Particularly preferred is e.g. an amino group wherein $R_{13}$ and $R_{14}$ are hydrogen atoms, an alkylamino group wherein $R_{13}$ is a hydrogen atom and $R_{14}$ is an alkyl group, or an arylamino group wherein $R_{13}$ is a hydrogen atom and $R_{14}$ is a phenyl group. The substituent which the alkyl group and the phenyl group may have may be a hydroxy group, a carboxy group, or a sulfo group.

Specific examples of the dye represented by the formula (5) may be those exemplified as the specific examples of the above formulae (1) to (4).

For preparation of an anisotropic dye film by a wet system film-forming method in the present invention, a known method such as a method may be employed wherein the dye composition for an anisotropic dye film is prepared and applied to a substrate such as a glass plate, and the dye is orientated and laminated.

Specifically, as the wet system film-forming method, a known method as disclosed in e.g. "Coating Engineering", Yuji Harasaki (Asakura Shoten K.K., published on March 20, 1971) pages 253-277 or "Creation and Applications of Harmonized Molecular Materials" supervised by Kunihiro Ichimura (CMC Publishing Co., Ltd., published on March 3, 1998) pages 118-149, or a method of coating a substrate preliminarily subjected to an alignment treatment by e.g. spin coating, spray coating, bar coating, roll coating, blade coating, free span coating or die coating, may be mentioned.

At the time of coating, the temperature is preferably at least 0°C and at most 80°C, and the humidity is preferably at least about 10%RH and at most about 80%RH. At the time of drying, the temperature is preferably at least 0°C and at most 120°C, and the humidity is preferably at least about 10%RH and at most about 80%RH.

As the substrate to be used in the present invention, glass, or a film of e.g. triacetate, acryl, polyester, triacetyl cellulose or urethane type may, for example, be mentioned. Further, on the surface of such a substrate, an alignment treatment layer may be applied by a known method as disclosed in e.g. "Ekisho Binran (Liquid Crystal Handbook)" (Maruzen Company, Limited, published on October 30, 2000) pages 226-239, in order to control the alignment direction of the dichroic dye.

The anisotropic dye film produced by such a method may have low mechanical strength in some cases, and thus a protective layer is provided if necessary. The protective layer is formed by lamination of a transparent polymer film such as a triacetate, acryl, polyester, polyimide, triacetyl cellulose or urethane type film and then subjected to practical use.

Further, in a case where the anisotropic dye film of the present invention is used as e.g. a polarizing filter for various display devices such as LCD or OLED, the dye film may be formed directly on e.g. an electrode substrate constituting such a display device, or a base material having the dye film formed thereon may be used as a constituting component of such a display device.

In a case where the anisotropic dye film is formed on a substrate by e.g. the above method, usually the thickness after drying is preferably at least 50 nm, more preferably at least 100 nm and preferably at most 50 $\mu$m, more preferably at most 1 $\mu$m.

The anisotropic dye film of the present invention will function as a polarizing film whereby a linearly polarized light, circularly polarized light or elliptically polarized light can be obtained by utilizing the anisotropy in light absorption. Further, it is capable of providing functions as various anisotropic films such as refractive anisotropy and conductivity anisotropy by selecting the film-forming process and the substrate or the composition containing the dye, whereby it can be made various types of polarizing elements which can be used for various purposes.

In a case where the anisotropic dye film of the present invention is formed on a substrate and used as a polarizing element, the formed anisotropic dye film itself may be used. Further, not only the above-mentioned protective layer but also layers having various functions such as an adhesive layer and an antireflection layer, an alignment film, and layers having optical functions such as a function as a retardation film, a function as a brightness enhancement film, a function as a reflection film, a function as a semi-transmissive reflective film, a function as a diffusion film and a function as an optical compensation film may be formed by lamination by e.g. a wet system film-forming method, so that it may be used in the form of a laminate.

Such layers having optical functions may be formed, for example, by the following methods.

A layer having a function as a retardation film may be formed by applying a stretching treatment as disclosed in e.g. Japanese Patent No. 2841377 or Japanese Patent No. 3094113, or by applying a treatment as disclosed in e.g. Japanese Patent No. 3168850.

Further, a layer having a function as a brightness enhancement film may be formed by forming ultrafine pores by a method as disclosed in e.g. JP-A-2002-169025 or JP-A-2003-29030, or by superposing two or more cholesteric liquid crystal layers with different central wavelengths of the selective reflection.

A layer having a function as a reflection film or a semi-transmissive reflection film may be formed by using a

metal thin film obtained by deposition or spattering.

A layer having a function as a diffusion film may be formed by coating the above protective layer with a resin solution containing fine particles.

Further, a layer having a function as a retardation film or an optical compensation film may be formed by applying a liquid crystalline compound such as a discotic liquid crystalline compound and orienting it.

EXAMPLES

Now, the present invention will be explained in further detail with reference to Examples. However, the present invention is by no means restricted to the following Examples within a range not to exceed the scope of the present invention.

In the following Examples, measurement regarding optical characteristics of the anisotropic dye film was carried out as follows.

(Dichroic ratio)

The dichroic ratio was obtained by measuring the transmittance of an anisotropic dye film by a spectrophotometer having an iodine type polarizing element disposed in an incident optical system, followed by calculation in accordance with the following formula:

$$\text{Dichroic ratio (D)} = Az/Ay$$

Az=-log(Tz)
Ay=-log(Ty)
Tz: Transmittance of a polarized light in the absorption axis direction of a dye film
Ty: Transmittance of a polarized light in the polarization axis direction of a dye film

(Chromaticity)

The chromaticities x and y (CIE 1964 supplementary standard colorimetric system, under standard illuminant $D_{65}$) of an anisotropic dye film were obtained by measuring the transmittance (single transmittance: Ts) in an unpolarized incident optical system by a spectrophotometer, followed by calculation in accordance with a method of JIS Z8701.

(Extinction properties)

As the extinction properties of an anisotropic dye film, the brightness (Y value) was calculated by measuring the transmittance (T right-angle) when two anisotropic dye films were disposed at right angles (they were superposed so that their polarization axes made right angles to each other) by a spectrophotometer, and introducing the measurement result into JIS Z8701 (1995) (CIE 1964 supplementary standard colorimetric system, under standard illuminant $D_{65}$).

EXAMPLE 1

26 parts of lithium salt of dye No. (1-1) was added to 74 parts of water and dissolved with stirring, followed by filtration to obtain a dye aqueous solution (a composition for an anisotropic dye film) having a pH of 7.

Separately, a glass substrate having a polyimide alignment film formed thereon by spin coating (75 mmx25 mm, thickness 1.1 mm, the polyimide alignment film with a polyimide film thickness of about 800 Å preliminarily subjected to rubbing treatment with cloth) was prepared. The above dye aqueous solution was applied to the substrate by an applicator with a gap of 10 μm (manufactured by Imoto Machinery Co., Ltd.), followed by air drying to obtain an anisotropic dye film.

Of the obtained anisotropic dye film, the dichroic ratio (D) obtained from the transmitted light (Tz) for a polarized light having a plane of vibration in the absorption axis direction in the plane of the dye film and the transmitted light (Ty) for a polarized light having a plane of vibration in the polarization axis direction in the plane of the dye film, and the maximum absorption wavelength (λmax) are shown in Table 1. The obtained anisotropic dye film had a high dichroic ratio (light absorption anisotropy) with which it can sufficiently function as a polarizing film.

EXAMPLE 2

37 parts of lithium salt of dye No. (1-2) was added to 63 parts of water and dissolved with stirring, followed by filtration to obtain a dye aqueous solution (a composition for an anisotropic dye film) having a pH of 7. The dye aqueous solution was applied to the same substrate as in Example 1 by an applicator with a gap of 2 μm (manufactured by Imoto Machinery Co., Ltd.), followed by air drying to obtain an anisotropic dye film.

The maximum absorption wavelength (λmax) and the dichroic ratio (D) of the obtained anisotropic dye film are shown in Table 1. The obtained anisotropic dye film was an anisotropic dye film having a high dichroic ratio with which it can sufficiently function as a polarizing film.

EXAMPLE 3

18 parts of sodium salt of dye No. (1-5) was added to 82 parts of water and dissolved with stirring, followed by filtration to obtain a composition for an anisotropic dye film having a pH of 7, which was applied under the same conditions as in Example 1, followed by air drying to obtain an anisotropic dye film.

The maximum absorption wavelength (λmax) and the dichroic ratio (D) of the obtained anisotropic dye film are shown in Table 1. The obtained anisotropic dye film was an anisotropic dye film having a high dichroic ratio with which it can sufficiently function as a polarizing film.

EXAMPLE 4

20 parts of sodium salt of dye No. (1-18) was added to 80 parts of water and dissolved with stirring, followed by filtration to obtain a composition for an anisotropic dye film having a pH of 7, which was applied under the same conditions as in Example 1, followed by air drying to obtain an anisotropic dye film.

The maximum absorption wavelength (λmax) and the dichroic ratio (D) of the obtained anisotropic dye film are shown in Table 1. The obtained anisotropic dye film was an anisotropic dye film having a high dichroic ratio with which it can sufficiently function as a polarizing film.

EXAMPLE 5

17 parts of sodium salt of dye No. (1-4) was added to 83 parts of water and dissolved with stirring, followed by filtration to obtain a composition for an anisotropic dye film having a pH of 7. An anisotropic dye film was obtained by application under the same conditions as in Example 1.

The maximum absorption wavelength (λmax) and the dichroic ratio (D) of the obtained anisotropic dye film are shown in Table 1. The obtained anisotropic dye film was an anisotropic dye film having a high dichroic ratio with which it can sufficiently function as a polarizing film.

EXAMPLE 6-1

0.1 Part of sodium salt of the above dye No. (1-1) was added to 99.9 parts of water and dissolved with stirring, followed by filtration to obtain a dye aqueous solution. The aqueous solution was injected into a quartz cell (cuvette) having an optical path length of 0.1 mm. The visible light transmittance (single transmittance: Ts) of each of the dye aqueous solution injected into the cuvette and the anisotropic dye film obtained in Example 1 was measured by a spectrophotometer to calculate the chromaticities x and y in the CIE 1964 supplementary standard colorimetric system under standard illuminant $D_{65}$.

Further, the chromaticity coordinates N of the standard illuminant $D_{65}$, and each of the chromaticity coordinates C1 of the obtained dye aqueous solution and chromaticity coordinates C2 of the anisotropic dye film, in the chromaticity diagram, were connected with a straight line, and wavelengths corresponding to the intersection points of extensions of the respective straight lines and the spectral locus were taken as the dominant wavelengths, and from the proportions at the respective points, the excitation purity (pe1) of the dye aqueous solution and the excitation purity (pe2) of the anisotropic dye film were calculated. The excitation purity of the dye aqueous solution and the excitation purity of the anisotropic dye film are shown in Table 2.

The excitation purity of the dye (dye aqueous solution) of the present Example was at most 12%. Further, the excitation purity of the anisotropic dye film prepared by using this dye was also at most 12%, and the anisotropic dye film was useful as a low chromaticness achromatic anisotropic dye film.

EXAMPLE 6-2

The excitation purities of the dyes used in Examples 2 to 5 and the anisotropic dye films obtained in Examples 2 to 5, were measured and calculated in the same manner as in Example 6. The excitation purities of the dye aqueous solutions and the excitation purities of the anisotropic dye films are shown in Table 2.

The excitation purities of the dyes (dye aqueous solutions) of the present Example were at most 12%. Further, the excitation purities of the anisotropic dye films prepared by using these dyes were also at most 12%, and the anisotropic dye films were useful as low chromaticness achromatic anisotropic dye films.

TABLE 1

| Ex. | Dye No. | Dichroic ratio | Wavelength (nm) | Concentration (%) | Coating method |
|---|---|---|---|---|---|
| 1 | 1-1 | 40 | 600 | 26 | Applicator (10 μm) |
| 2 | 1-2 | 25 | 600 | 37 | Applicator (2 μm) |
| 3 | 1-5 | 33 | 580 | 18 | Applicator (10 μm) |
| 4 | 1-18 | 17 | 540 | 20 | Applicator (10 μm) |
| 5 | 1-4 | 13 | 590 | 17 | Applicator (10 μm) |

TABLE 2

| Ex. | Dye No. | Excitation purity (pe1) | Excitation purity (pe2) |
|---|---|---|---|
| 1 | 1-1 | 10.5% | 9.1% |
| 2 | 1-2 | 5.7% | 12.0% |
| 3 | 1-5 | 4.6% | 9.5% |
| 4 | 1-18 | 4.1% | 8.8% |
| 5 | 1-4 | 4.1% | 6.7% |

COMPARATIVE EXAMPLE 1

43 parts of sodium salt of the following (II-1) was added to 57 parts of water and dissolved with stirring, followed by filtration to obtain a composition for an anisotropic dye film having a pH of 7, which was applied in the same manner as in Example 2, followed by air drying to obtain an anisotropic dye film.

The obtained dye film was subjected to various tests in the same manner as in Example 1. The results are shown in Table 3. The obtained dye film had a dichroic ratio (absorption anisotropy) of 4, and did not provide adequate anisotropy.

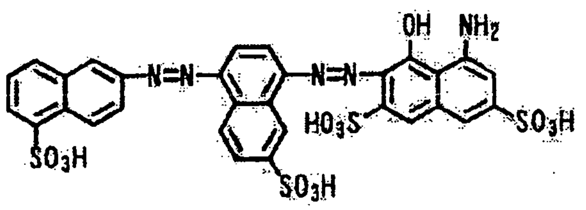

II-1

COMPARATIVE EXAMPLE 2

A composition for an anisotropic dye film was prepared in the same manner as in Comparative Example 1 except that sodium salt of dye (II-2) was used instead of the dye (II-1), and the composition was applied to the same substrate under the same conditions to obtain a dye film.

The obtained dye film was subjected to various tests in the same manner as in Example 1. The results are shown in Table 3. The obtained dye film had a dichroic ratio (absorption anisotropy) of at most 2, and did not provide

adequate anisotropy.

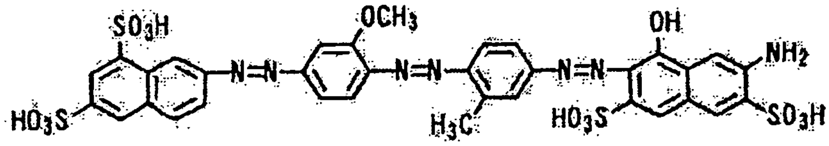

II-2

COMPARATIVE EXAMPLE 3

10 parts of sodium salt of dye (II-3) was added to 90 parts of water and dissolved with stirring, followed by filtration to obtain a composition for an anisotropic dye film having a pH of 7, which was applied to the same substrate as in Example 1 by a bar coater No. 3 by TESTER SANGYO CO., LTD. to obtain a dye film.

The obtained dye film was subjected to various tests in the same manner as in Example 1. The results are shown in Table 3. The obtained dye film had a dichroic ratio (absorption anisotropy) of 2, and did not provide adequate anisotropy.

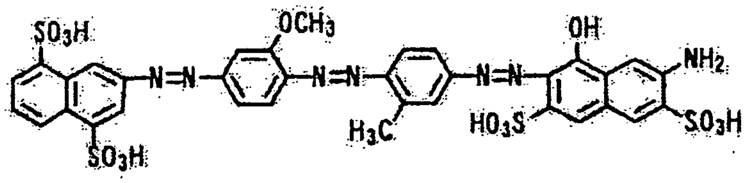

II-3

TABLE 3

| Comp. Ex. | Dye No. | Dichroic ratio | Wavelength (nm) | Concentration (%) | Coating method |
|---|---|---|---|---|---|
| 1 | II-1 | 4 | 580 | 43 | Applicator (2 μm) |
| 2 | II-2 | <2 | 600 | 10 | Bar coater (#3) |
| 3 | II-3 | 2 | 580 | 10 | Bar coater (#3) |

EXAMPLE 7

18 parts of lithium salt of dye No. 3-30 (exemplified compound (3-30)) was added to 82 parts of water and dissolved with stirring, followed by filtration to obtain a dye aqueous solution (a composition for an anisotropic dye film) having a pH of 7.

Separately, the same substrate as in Example 1 was prepared. The dye aqueous solution was applied to the substrate by an applicator with a gap of 10 μm (manufactured by Imoto Machinery Co., Ltd.), followed by air drying to obtain an anisotropic dye film.

Of the obtained anisotropic dye film, the transmitted light (Tz) for a polarized light having a plane of vibration in the absorption axis direction in the plane of the dye film and the transmitted light (Ty) for a polarized light having a plane of vibration in the polarization axis direction in the plane of the dye film were measured, and the dichroic ratio (D) was calculated therefrom. The anisotropic dye film of the present Example 7 had a dichroic ratio of 18.2 (705 nm) and had a high dichroic ratio (light absorption anisotropy) with which it can sufficiently function as a polarizing film.

Further, two substrates provided with an anisotropic dye film were prepared in the same manner as above, and they were superposed at right angles to measure the right-angle transmittance (T right-angle), and then the brightness (Y value) was calculated. The brightness was so small as 0.086 when the dye films were disposed at right angles, which indicates excellent shading properties, and it was confirmed that the anisotropic dye film can function as a polarizing film with high extinction properties.

EXAMPLE 8

20 parts of lithium salt of dye No. (3-27) (exemplified compound (3-27)) was added to 80 parts of water and dissolved with stirring, followed by filtration to obtain a dye aqueous solution (a composition for an anisotropic dye film) having a pH of 7, which was applied to the same substrate as in Example 1 in the same manner, followed by air drying to obtain an anisotropic dye film.

The obtained anisotropic dye film had a dichroic ratio (D) of 51.6 (695 nm) as calculated from measured values of the transmitted light (Tz) and (Ty), and was an anisotropic dye film having a high dichroic ratio with which it can sufficiently function as a polarizing film.

Further, the right-angle transmittance (T right-angle) was measured in the same manner as in Example 7, and the brightness when the dye films were disposed at right angles was calculated and as a result, it was so small as 0.296, which indicates excellent shading properties, and it was confirmed that the anisotropic dye film can function as a polarizing film with high extinction properties.

EXAMPLE 9

10 parts of sodium salt of the above dye No. (3-30) was added to 90 parts of water and dissolved with stirring, followed by filtration to obtain a dye aqueous solution. The aqueous solution was injected into a quartz cell (cuvette) having an optical path length of 0.01 mm. The visible light transmittance of the dye aqueous solution injected into the cuvette was measured by a spectrophotometer to calculate the molar extinction coefficient (ε) at the maximum absorption wavelength at a dye concentration of 10 wt%. Further, with respect to sodium salt of dye No. 3-27, the molar extinction coefficient (ε) at a dye concentration of 10 wt% was calculated in the same manner.

Then, 0.1 part of sodium salt of dye No. (3-30) was added to 99.9 parts of water and dissolved with stirring, followed by filtration to obtain a dye aqueous solution. The aqueous solution was injected into a cuvette having an optical path length of 0.1 mm. The visible light transmittance of the dye aqueous solution injected into the cuvette was measured by a spectrophotometer to calculate the molar extinction coefficient (ε) at a dye concentration of 1,000 ppm. Further, with respect to sodium salt of dye No. 3-27, the molar extinction coefficient (ε) at a dye concentration of 1,000 ppm was calculated in the same manner.

Further, 0.001 part of sodium salt of dye No. (3-30) was added to 99.999 parts of water and dissolved with stirring, followed by filtration to obtain a dye aqueous solution. The aqueous solution was injected into a cuvette having an optical path length of 10 mm. The visible light transmittance of the dye aqueous solution injected into the cuvette was measured by a spectrophotometer to calculate the molar extinction coefficient (ε) at a dye concentration of 10 ppm. Further, with respect to sodium salt of dye No. 3-27, the molar extinction coefficient (e) at a dye concentration of 10 ppm was calculated in the same manner.

From the above results, the relation between the dye concentration in the solution and the molar extinction coefficient is shown in Table 4 and Fig. 1. The dye for an anisotropic dye film of the present invention was confirmed that the degree of lightening (decrease in the molar extinction coefficient) along with the increase of the dye concentration in the solution (composition) tends to be low, and that the dye film obtained by coating and drying has high shading properties.

TABLE 4

| Dye concentration (wt%) | Molar extinction coefficient (ε) | |
|---|---|---|
| | Compound No. (3-27) | Compound No. (3-30) |
| 10 | 21,906 | 25,916 |
| 0.1 | 30,543 | 36,092 |
| 0.001 | 32,789 | 36,189 |

EXAMPLE 10

0.1 part of lithium salt of the above dye No. (3-30) was added to 99.9 parts of water and dissolved with stirring, followed by filtration to obtain a dye aqueous solution. The aqueous solution was injected into a quartz cell (cuvette) having an optical path length of 0.1 mm. The visible light transmittance (single transmittance: Ts) of each of the dye aqueous solution injected into the cuvette and the anisotropic dye film obtained in Example 7 was measured by a spectrophotometer to calculate the chromaticities x and y in the CIE 1964 supplementary standard colorimetric system under standard illuminant $D_{65}$.

Further, the chromaticity coordinates N of the standard illuminant $D_{65}$, and each of the chromaticity coordinates C1 of the obtained dye aqueous solution and chromaticity coordinates C2 of the anisotropic dye film, in the chromaticity diagram, were connected with a straight line, and wavelengths corresponding to the intersection points of extensions of the respective straight lines and the spectral locus were taken as the dominant wavelengths, and from the proportions at the respective points, the excitation purity (pe1) of the dye aqueous solution and the excitation purity (pe2) of the anisotropic dye film were calculated. The excitation purity of the dye aqueous solution and the excitation purity of the anisotropic dye film are shown in Table 5.

The excitation purity of the dye (dye aqueous solution) of the present Example was at most 12%. Further, the excitation purity of the anisotropic dye film prepared by using this dye was also at most 12%, and the anisotropic dye film was useful as a low chromaticness achromatic anisotropic dye film.

EXAMPLE 11

The excitation purities of the dye used in Example 8 and the anisotropic dye film obtained in Example 8 were measured and calculated in the same manner as in Example 10. The excitation purity of the dye aqueous solution and the excitation purity of the anisotropic dye film are shown in Table 5.

The excitation purity of the dye (dye aqueous solution) of the present Example was at most 12%. Further, the excitation purity of the anisotropic dye film prepared by using the dye was also at most 16%, and the anisotropic dye film was useful as a low chromaticness achromatic anisotropic dye film. Further, the anisotropic dye film had a high dichroic ratio with which it can sufficiently function as a polarizing film.

TABLE 5

| Ex. | Dye No. | Excitation purity (pe1) | Excitation purity (pe2) |
|---|---|---|---|
| 7 | 3-30 | 6% | 12% |
| 8 | 3-27 | 6% | 16% |

EXAMPLE 12

18 parts of lithium salt of dye No. (4-1) was added to 82 parts of water and dissolved with stirring, followed by filtration to obtain a dye aqueous solution (a composition for an anisotropic dye film) having a pH of 7.

Separately, the same substrate as in Example 1 was prepared, to which above dye aqueous solution was applied by an applicator with a gap of 10 μm (manufactured by Imoto Machinery Co., Ltd.), followed by air drying to obtain an anisotropic dye film.

Of the obtained anisotropic dye film, the dichroic ratio (D) obtained from the transmitted light (Tz) for a polarized light having a plane of vibration in the absorption axis direction in the plane of the dye film and the transmitted light (Ty) for a polarized light having a plane of vibration in the polarization axis direction in the plane of the dye film, and the maximum absorption wavelength (λmax) are shown in Table 6. The obtained anisotropic dye film had a high dichroic ratio (light absorption anisotropy) with which it can sufficiently function as a polarizing film.

EXAMPLE 13

14 parts of lithium salt of dye No. (4-2) was added to 86 parts of water and dissolved with stirring, followed by filtration to obtain a dye aqueous solution (a composition for an anisotropic dye film) having a pH of 7.

The aqueous solution was applied under the same conditions as in Example 12, followed by air drying to obtain an anisotropic dye film.

The maximum absorption wavelength (λmax) and the dichroic ratio (D) of the obtained anisotropic dye film are shown in Table 6. The obtained anisotropic dye film was an anisotropic dye film having a high dichroic ratio with which it can sufficiently function as a polarizing film.

EXAMPLE 14

10 parts of lithium salt of dye No. (4-3) was added to 90 parts of water and dissolved with stirring, followed by filtration to obtain a composition for an anisotropic dye film having a pH of 7.

The dye aqueous solution was applied to the same substrate as in Example 1 by an applicator with a gap of 20 μm (manufactured by Imoto Machinery Co., Ltd.), followed by air drying to obtain an anisotropic dye film.

The maximum absorption wavelength (λmax) and the dichroic ratio (D) of the obtained anisotropic dye film are

shown in Table 6. The obtained anisotropic dye film was an anisotropic dye film having a high dichroic ratio with which it can sufficiently function as a polarizing film.

EXAMPLE 15

12 parts of lithium salt of dye No. (4-5) was added to 88 parts of water and dissolved with stirring, followed by filtration to obtain a composition for an anisotropic dye film having a pH of 7.

The composition was applied under the same conditions as in Example 12, followed by air drying to obtain an anisotropic dye film.

The maximum absorption wavelength (λmax) and the dichroic ratio (D) of the obtained anisotropic dye film are shown in Table 6. The obtained anisotropic dye film was an anisotropic dye film having a high dichroic ratio with which it can sufficiently function as a polarizing film.

EXAMPLE 16

10 parts of lithium salt of the above dye No. (4-1) and 10 parts of lithium salt of the above dye No. (4-2) were added to 80 parts of water and dissolved with stirring, followed by filtration to obtain a composition for an anisotropic dye film having a pH of 7.

The dye aqueous solution was applied to the same substrate as in Example 1 by an applicator with a gap of 20 μm (manufactured by Imoto Machinery Co., Ltd.), followed by air drying to obtain an anisotropic dye film.

The maximum absorption wavelength (λmax) and the dichroic ratio (D) of the obtained anisotropic dye film are shown in Table 6. The obtained anisotropic dye film was an anisotropic dye film having a high dichroic ratio with which it can sufficiently function as a polarizing film.

EXAMPLE 17

0.1 part of lithium salt of the above dye No. (4-1) was added to 99.9 parts of water and dissolved with stirring, followed by filtration to obtain a dye aqueous solution. The aqueous solution was injected into a quartz cell (cuvette) having an optical path length of 0.1 mm. The visible light transmittance (single transmittance: Ts) of each of the dye aqueous solution injected into the cuvette and the anisotropic dye film obtained in Example 12 was measured by a spectrophotometer to calculate the chromaticities x and y in the CIE 1964 supplementary standard colorimetric system under standard illuminant $D_{65}$.

Further, the chromaticity coordinates N of the standard illuminant $D_{65}$, and each of the chromaticity coordinates C1 of the obtained dye aqueous solution and chromaticity coordinates C2 of the anisotropic dye film, in the chromaticity diagram, were connected with a straight line, and wavelengths corresponding to the intersection points of extensions of the respective straight lines and the spectral locus were taken as the dominant wavelengths, and from the proportions at the respective points, the excitation purity (pe1) of the dye aqueous solution and the excitation purity (pe2) of the anisotropic dye film were calculated. The excitation purity of the dye aqueous solution and the excitation purity of the anisotropic dye film are shown in Table 7.

The excitation purity of the dye (dye aqueous solution) of the present Example was at most 12%. Further, the excitation purity of the anisotropic dye film prepared by using this dye was also at most 12%, and the anisotropic dye film was useful as a low chromaticness achromatic anisotropic dye film.

EXAMPLE 18

The excitation purities of the dye used in Example 13 and the anisotropic dye film obtained in Example 13 were measured and calculated in the same manner as in Example 17. The excitation purity of the dye aqueous solution and the excitation purity of the anisotropic dye film are shown in Table 7.

EXAMPLE 19

The excitation purities of the dye used in Example 14 and the anisotropic dye film obtained in Example 14 were measured and calculated in the same manner as in Example 17. The excitation purity of the dye aqueous solution and the excitation purity of the anisotropic dye film are shown in Table 7.

EXAMPLE 20

The excitation purities of the dye used in Example 15 and the anisotropic dye film obtained in Example 15 were

measured and calculated in the same manner as in Example 17. The excitation purity of the dye aqueous solution and the excitation purity of the anisotropic dye film are shown in Table 7.

EXAMPLE 21

The excitation purity of the anisotropic dye film obtained in Example 16 was measured and calculated in the same manner as in Example 17. The excitation purity of the anisotropic dye film is shown in Table 7.

The excitation purities of the dyes (dye aqueous solutions) in Examples 18 to 20 were at most 12%. Further, the excitation purities of anisotropic dye films prepared by using the dyes and the excitation purity of the anisotropic dye film obtained in Example 16 were also at most 12, and the anisotropic dye films were found to be useful as low chromaticness achromatic anisotropic dye films.

TABLE 6

| Ex. | Dye No. | Maximum absorption wavelength | Dichroic ratio |
|---|---|---|---|
| | | (λmax) | (D) |
| 12 | (4-1) | 625 | 24 |
| 13 | (4-2) | 595 | 23 |
| 14 | (4-3) | 605 | 37 |
| 15 | (4-5) | 620 | 45 |
| 16 | (4-1), (4-2) | 610 | 25 |

TABLE 7

| Ex. | Excitation purity of dye aqueous solution | Excitation purity of anisotropic dye film |
|---|---|---|
| | (pe1) | (pe2) |
| 17 | 5.7% | 12.0% |
| 18 | 5.4% | 7.0% |
| 19 | 5.1% | 5.8% |
| 20 | 4.1% | 8.3% |
| 21 | - | 10.1% |

EXAMPLE 22

6 parts of lithium salt of dye No. (4-22) was added to 94 parts of water and dissolved with stirring, followed by filtration to obtain a dye aqueous solution (a composition for an anisotropic dye film) having a pH of 7.

Separately, the same substrate as in Example 1 was prepared, to which the above dye aqueous solution was applied by an applicator with a gap of 30 μm (manufactured by Imoto Machinery Co., Ltd.), followed by air drying to obtain an anisotropic dye film.

Of the obtained anisotropic dye film, the dichroic ratio (D) obtained from the transmitted light (Tz) for a polarized light having a plane of vibration in the absorption axis direction in the plane of the dye film and the transmitted light (Ty) for a polarized light having a plane of vibration in the polarization axis direction in the plane of the dye film, and the maximum absorption wavelength (λmax) are shown in Table 8. The obtained anisotropic dye film had a high dichroic ratio (light absorption anisotropy) with which it can sufficiently function as a polarizing film.

EXAMPLE 23

25 parts of lithium salt of dye No. (3-45) was added to 75 parts of water and dissolved with stirring, followed by filtration to obtain a dye aqueous solution (a composition for an anisotropic dye film) having a pH of 7.

Separately, the same substrate as in Example 1 was prepared, to which the dye aqueous solution was applied by an applicator with a gap of 5 μm (manufactured by Imoto Machinery Co., Ltd.), followed by air drying to obtain an

anisotropic dye film.

The maximum absorption wavelength (λmax) and the dichroic ratio (D) of the obtained anisotropic dye film are shown in Table 8. The obtained anisotropic dye film was an anisotropic dye film having a high dichroic ratio with which it can sufficiently function as a polarizing film.

EXAMPLE 24

16 parts of lithium salt of dye No. (3-35) was added to 84 parts of water and dissolved with stirring, followed by filtration to obtain a dye aqueous solution (a composition for an anisotropic dye film) having a pH of 7.

The dye aqueous solution was applied to the same substrate as in Example 1 by an applicator with a gap of 10 μm (manufactured by Imoto Machinery Co., Ltd.), followed by air drying to obtain an anisotropic dye film.

The maximum absorption wavelength (λmax) and the dichroic ratio (D) of the obtained anisotropic dye film are shown in Table 8. The obtained anisotropic dye film was an anisotropic dye film having a high dichroic ratio with which it can sufficiently function as a polarizing film.

EXAMPLE 25

13 parts of lithium salt of dye No. (3-32) was added to 87 parts of water and dissolved with stirring, followed by filtration to obtain a dye aqueous solution (a composition for an anisotropic dye film) having a pH of 7.

The dye aqueous solution was applied to the same substrate as in Example 1 by a bar coater (manufactured by TESTER SANGYO CO., LTD., No.2), followed by air drying to obtain an anisotropic dye film.

The maximum absorption wavelength (λmax) and the dichroic ratio (D) of the obtained anisotropic dye film are shown in Table 8. The obtained anisotropic dye film was an anisotropic dye film having a high dichroic ratio with which it can sufficiently function as a polarizing film.

EXAMPLE 26

14 parts of lithium salt of dye No. (1-22) was added to 86 parts of water and dissolved with stirring, followed by filtration to obtain a dye aqueous solution (a composition for an anisotropic dye film) having a pH of 7.

The dye aqueous solution was applied to the same substrate as in Example 1 by an applicator with a gap of 10 μm (manufactured by Imoto Machinery Co., Ltd.), followed by air drying to obtain an anisotropic dye film.

The maximum absorption wavelength (λmax) and the dichroic ratio (D) of the obtained anisotropic dye film are shown in Table 8. The obtained anisotropic dye film was an anisotropic dye film having a high dichroic ratio with which it can sufficiently function as a polarizing film.

EXAMPLE 27

22 parts of lithium salt of dye No. (4-24) was added to 78 parts of water and dissolved with stirring, followed by filtration to obtain a dye aqueous solution (a composition for an anisotropic dye film) having a pH of 7.

The dye aqueous solution was applied to a slide glass ("Colorless edge polish frosted slide glass No. 1" manufactured by MATSUNAMI GLASS IND., LTD.) by an applicator with a gap of 10 μm (manufactured by Imoto Machinery Co., Ltd.) followed by air drying to obtain an anisotropic dye film.

The maximum absorption wavelength (λmax) and the dichroic ratio (D) of the obtained anisotropic dye film are shown in Table 8. The obtained anisotropic dye film was an anisotropic dye film having a high dichroic ratio with which it can sufficiently function as a polarizing film.

TABLE 8

| Ex. | Dye No. | Maximum absorption wavelength (nm) | Dichroic ratio (D) |
|---|---|---|---|
| 22 | (4-22) | 610 | 20 |
| 23 | (3-45) | 585 | 20 |
| 24 | (3-35) | 580 | 33 |
| 25 | (3-32) | 570 | 30 |
| 26 | (1-22) | 580 | 14 |

(continued)

| Ex. | Dye No. | Maximum absorption wavelength (nm) | Dichroic ratio (D) |
|---|---|---|---|
| 27 | (4-24) | 585 | 23 |

EXAMPLE 28

0.1 Part of lithium salt of the above dye No. (4-22) was added to 99.9 parts of water and dissolved with stirring, followed by filtration to obtain a dye aqueous solution. The aqueous solution was injected into a quartz cell (cuvette) having an optical path length of 0.1 mm. The visible light transmittance (single transmittance: Ts) of each of the dye aqueous solution injected into the cuvette and the anisotropic dye film obtained in Example 22 was measured by a spectrophotometer to calculate the chromaticities x and y in the CIE 1964 supplementary standard colorimetric system under standard illuminant $D_{65}$.

Further, the chromaticity coordinates N of the standard illuminant $D_{65}$, and each of the chromaticity coordinates C1 of the obtained dye aqueous solution and chromaticity coordinates C2 of the anisotropic dye film, in the chromaticity diagram, were connected with a straight line, and wavelengths corresponding to the intersection points of extensions of the respective straight lines and the spectral locus were taken as the dominant wavelengths, and from the proportions at the respective points, the excitation purity (pe1) of the dye aqueous solution and the excitation purity (pe2) of the anisotropic dye film were calculated. The excitation purity of the dye aqueous solution and the excitation purity of the anisotropic dye film are shown in Table 9.

The excitation purity of the dye (dye aqueous solution) of the present Example was at most 12%. Further, the excitation purity of the anisotropic dye film prepared by using this dye was also at most 12%, and the anisotropic dye film was useful as a low chromaticness achromatic anisotropic dye film.

EXAMPLE 29

The excitation purities of the dyes used in Examples 23 to 27 and the anisotropic dye films obtained in Examples 23 to 27, were measured and calculated in the same manner as in Example 28. The excitation purities of the dye aqueous solutions and the excitation purities of the anisotropic dye films are shown in Table 9. The excitation purities of the dyes (dye aqueous solutions) of the present Example were at most 12%. Further, the excitation purities of the anisotropic dye films prepared by using these dyes were also at most 12%, and the anisotropic dye films were found to be useful as low chromaticness achromatic anisotropic dye films.

TABLE 9

| Ex. | Dye No. | Excitation purity (pe1) | Excitation purity (pe2) |
|---|---|---|---|
| 22 | (4-22) | 10% | 6% |
| 23 | (3-45) | 5% | 10% |
| 24 | (3-35) | 7% | 8% |
| 25 | (3-32) | 3% | 11% |
| 26 | (1-22) | 5% | 12% |
| 27 | (4-24) | 5% | 8% |

INDUSTRIAL APPLICABILITY

Employing a dye for an anisotropic dye film which is achromatic and has high dichroism and a high degree of molecular orientation, an anisotropic dye film which also is achromatic and has high dichroism and a high degree of molecular orientation, can be provided. A polarizing element prepared by employing the anisotropic dye film is useful for e.g. polarizing plates provided on display devices such as light control devices, liquid crystal devices (LCD) and organic electroluminescence devices (OLED).

The entire disclosures of Japanese Patent Application No. 2005-107636 filed on April 4, 2005, Japanese Patent Application No. 2005-110535 filed on April 7, 2005, Japanese Patent Application No. 2005-123092 filed on April 21, 2005, Japanese Patent Application No. 2005-295499 filed on October 7, 2005 and Japanese Patent Application No.

2006-084605 filed on March 27, 2006 including specifications, claims, drawings and summaries are incorporated herein by reference in their entireties.

## Claims

1. A dye for an anisotropic dye film to be formed by a wet system film-forming method, of which the free acid form is represented by the following formula (I):

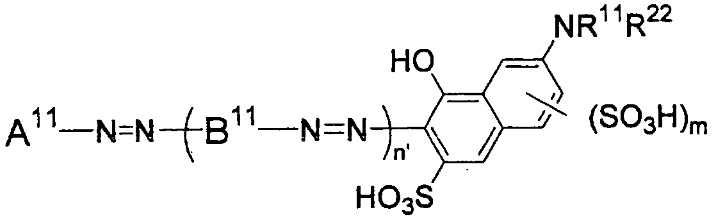

(I)

wherein each of $R^{11}$ and $R^{22}$ which are independent of each other, is a hydrogen atom, an alkyl group which may have a substituent, a phenyl group which may have a substituent, or an acyl group which may have a substituent, $A^{11}$ is a phenyl group which may have a substituent, a naphthyl group which may have a substituent, or an aromatic heterocyclic group which may have a substituent,
$B^{11}$ is a bivalent aromatic hydrocarbon group which may have a substituent, or a bivalent aromatic heterocyclic group which may have a substituent,
m is 0 or 1, and n' is 1 or 2, provided that when n' is 1, $A^{11}$ is not a phenyl group having a vinyl group, and when n' is 2, $A^{11}$ is a group of the following formula (I-a) or (I-b) or an aromatic heterocyclic group which may have a substituent:

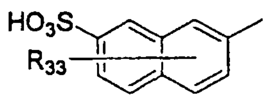 (I-a) 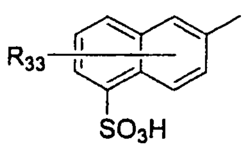 (I-b)

(wherein $R_{33}$ is a hydrogen atom, a hydroxy group or an alkoxy group which may have a substituent), provided that when n' is 2, a plurality of $B^{11}$ in one molecule may be the same or different.

2. The dye for an anisotropic dye film according to Claim 1, of which the free acid form is represented by the following formula (1):

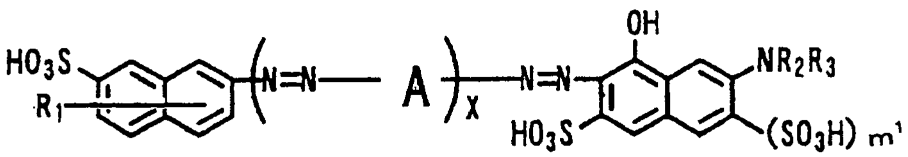

(1)

wherein A is a phenylene group which may have a substituent, or a naphthylene group which may have a substituent; $R_1$ is a hydrogen atom, a hydroxy group or an alkoxy group which may have a substituent; each of $R_2$ and $R_3$ which are independent of each other, is a hydrogen atom, an alkyl group which may have a substituent, a phenyl group which may have a substituent, or an acyl group which may have a substituent; $m^1$ is 0 or 1; and X is 1 or 2; provided that when X is 2, a plurality of A in one molecule may be the same or different.

3. The dye for an anisotropic dye film according to Claim 1, of which the free acid form is represented by the following formula (2):

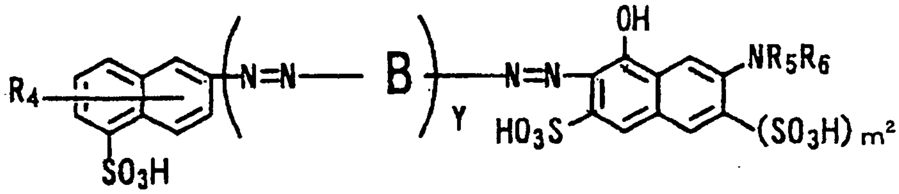

(2)

wherein B is a phenylene group which may have a substituent, or a naphthylene group which may have a substituent; $R_4$ is a hydrogen atom, a hydroxy group or an alkoxy group which may have a substituent; each of $R_5$ and $R_6$ is a hydrogen atom, an alkyl group which may have a substituent, a phenyl group which may have a substituent, or an acyl group which may have a substituent; $m^2$ is 0 or 1; and Y is 1 or 2; provided that when Y is 2, a plurality of B in one molecule may be the same or different.

4. The dye for an anisotropic dye film according to Claim 1, of which the free acid form is represented by the following formula (3):

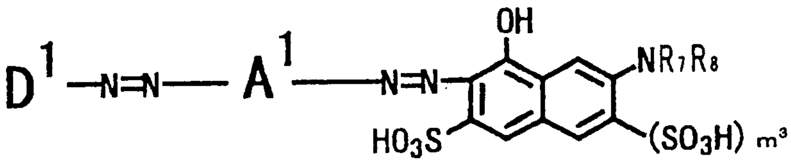

(3)

wherein $D^1$ is a phenyl group which may have a substituent other than a vinyl group, a naphthyl group which may have a substituent, or an aromatic heterocyclic group which may have a substituent; $A^1$ is an aromatic hydrocarbon group which may have a substituent,
each of $R_7$ and $R_8$ which are independent of each other, is a hydrogen atom, an alkyl group which may have a substituent, a phenyl group which may have a substituent, or an acyl group which may have a substituent; and $m^3$ is 0 or 1.

5. The dye for an anisotropic dye film according to Claim 4, wherein in the above formula (3), $D^1$ is a phenyl group which may have a substituent other than a vinyl group, a naphthyl group which may have a substituent, or an aromatic heterocyclic group which may have a substituent, and the substituent is a group having polarity.

6. The dye for an anisotropic dye film according to Claim 4 or 5, wherein in the above formula (3), $D^1$ is a phenyl group which may have a substituent other than a vinyl group, or a naphthyl group which may have a substituent.

7. The dye for an anisotropic dye film according to any ones of Claims 4 to 6, wherein in the above formula (3), $A^1$ is a phenylene group which may have a substituent, or a naphthylene group which may have a substituent.

8. The dye for an anisotropic dye film according to Claim 1, of which the free acid form is represented by the following formula (4):

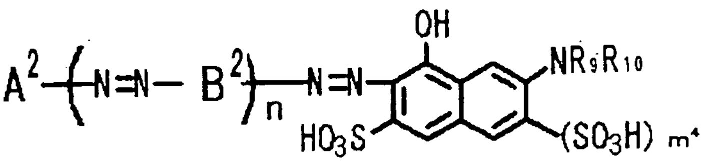

(4)

wherein $A^2$ is any of groups of the following formulae (4-a), (4-b) and (4-c), which may have a substituent:

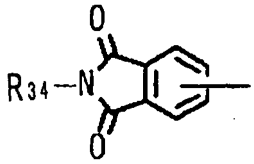

(4 − a)

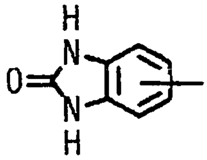

(4 − b)

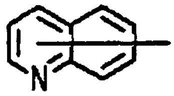

(4 − c)

(wherein $R_{34}$ is a hydrogen atom, an alkyl group which may have a substituent, or a phenyl group which may have a substituent),
$B^2$ is a bivalent aromatic hydrocarbon group which may have a substituent, or a bivalent aromatic heterocyclic group containing as the hetero atom a nitrogen atom which may have a substituent; each of $R_9$ and $R_{10}$ which are independent of each other, is a hydrogen atom, an alkyl group which may have a substituent, a phenyl group which may have a substituent, or an acyl group which may have a substituent; $m^4$ is 0 or 1; and n is 1 or 2;
provided that when n is 2, a plurality of $B^2$ in one molecule may be the same or different.

**9.** The dye for an anisotropic dye film according to Claim 8, wherein in the above formula (4), $A^2$ is any of groups of the following formulae (4-a1), (4-b1), (4-c1) and (4-c2), which may have a substituent:

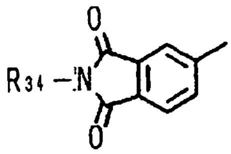

(4 − a 1)

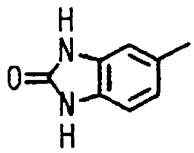

(4 − b 1)

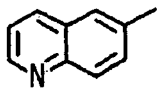

(4 − c 1)

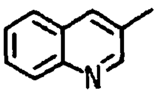

(4 − c 2)

wherein $R_{34}$ is a hydrogen atom, an alkyl group which may have a substituent, or a phenyl group which may have a substituent.

**10.** The dye for an anisotropic dye film according to Claim 8 or 9, wherein the dye represented by the above formula (4) is water soluble.

**11.** The dye for an anisotropic dye film according to any one of Claims 1 to 10, which has an excitation purity of from 0% to 12%.

**12.** A composition for an anisotropic dye film, which contains the dye for an anisotropic dye film as defined in any one of Claims 1 to 11 and a solvent.

**13.** An anisotropic dye film, which contains the dye for an anisotropic dye film as defined in any one of Claims 1 to 11.

**14.** The anisotropic dye film according to Claim 13 containing the dye for an anisotropic dye film as defined in any one of Claims 1 to 11, which is formed by a wet system film-forming method.

**15.** The anisotropic dye film according to Claim 14 containing the dye for an anisotropic dye film as defined in any one of Claims 1 to 11 and formed by a wet system film-forming method, which has a dichroic ratio of at least 15.

**16.** An anisotropic dye film containing a dye, of which the free acid form is represented by the following formula (5), formed by a wet system film-forming method, and having a dichroic ratio of at least 40:

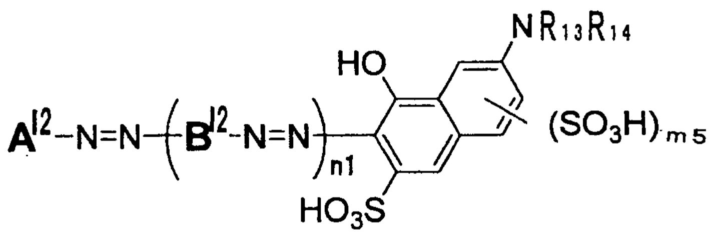

(5)

wherein $A^{12}$ is an aromatic hydrocarbon group which may have a substituent, or an aromatic heterocyclic group which may have a substituent; $B^{12}$ is a bivalent aromatic hydrocarbon group which may have a substituent, or a bivalent aromatic heterocyclic group which may have a substituent; each of $R_{13}$ and $R_{14}$ which are independent of each other, is a hydrogen atom, an alkyl group which may have a substituent, a phenyl group which may have a substituent, or an acyl group which may have a substituent; $m^5$ is 0 or 1; and n1 is 1 or 2;
provided that when n1 is 2, a plurality of $B^{12}$ in one molecule may be the same or different.

**17.** A polarizing element employing the anisotropic dye film as defined in any one of Claims 13 to 16.

**18.** An azo dye, of which the free acid form is represented by the following formula (6):

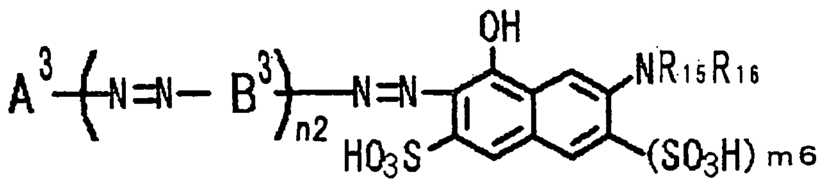

(6)

wherein $A^3$ is any of groups of the following formulae (6-a), (6-b) and (6-c), which may have a substituent:

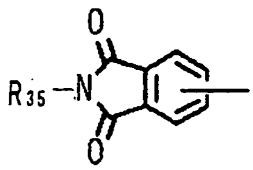

(6-a)

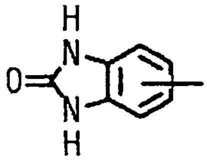

(6-b)

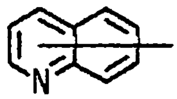

(6-c)

(wherein $R_{35}$ is a hydrogen atom, an alkyl group which may have a substituent, or a phenyl group which may have a substituent),

$B^3$ is a bivalent aromatic hydrocarbon group which may have a substituent, or a bivalent aromatic heterocyclic group containing as the hetero atom a nitrogen atom which may have a substituent; each of $R_{15}$ and $R_{16}$ which are independent of each other, is a hydrogen atom, an alkyl group which may have a substituent, a phenyl group which may have a substituent, or an acyl group which may have a substituent;

$m^6$ is 0 or 1, and n2 is 1 or 2;

provided that when n2 is 2, a plurality of $B^3$ in one molecule may be the same or different.

**19.** The azo dye according to Claim 18, wherein in the above formula (6), $A^3$ is any of groups of the following formulae (6-a1), (6-b1), (6-c1) and (6-c2), which may have a substituent:

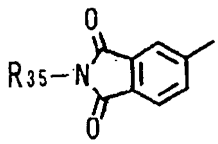

(6-a1)

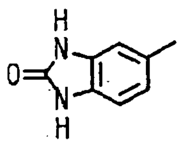

(6-b1)

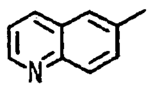

(6-c1)

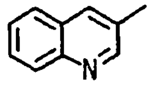

(6 − c 2)

wherein $R_{35}$ is a hydrogen atom, an alkyl group which may have a substituent, or a phenyl group which may have a substituent.

**20.** The azo dye according to Claim 18 or 19, wherein the azo dye represented by the above formula (6) is water soluble.

Fig. 1

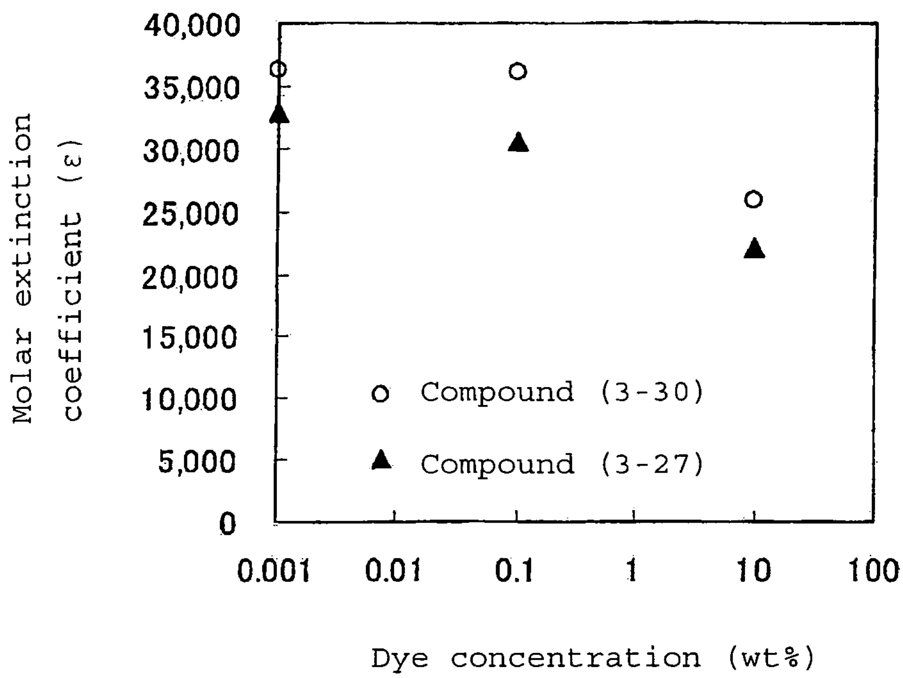
40,000
35,000
30,000
25,000
20,000
15,000
10,000
5,000
0
Molar extinction coefficient (ε)
Compound (3-30)
Compound (3-27)
0.001
0.01
0.1
1
10
100
Dye concentration (wt%)

**INTERNATIONAL SEARCH REPORT**

International application No.
PCT/JP2006/307080

A. CLASSIFICATION OF SUBJECT MATTER
*C09B31/08*(2006.01), *C07D209/48*(2006.01), *C07D215/38*(2006.01), *C07D235/26*(2006.01), *C07D277/66*(2006.01), *C09B31/22*(2006.01), *C09D201/00*(2006.01), *G02B5/30*(2006.01)
According to International Patent Classification (IPC) or to both national classification and IPC

B. FIELDS SEARCHED

Minimum documentation searched (classification system followed by classification symbols)
C07D209/48, C07D215/38, C07D235/26, C07D277/66, C09B31/08, C09B31/22, C09D201/00, G02B5/30

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>A | JP 55-90926 A (Nitto Electric Industrial Co., Ltd.),<br>10 July, 1980 (10.07.80),<br>Structural formula (A)<br>(Family: none) | 1,4-7,11-17<br>2,3,8-10,<br>18-20 |
| X<br>A | JP 8-67824 A (Mitsui Toatsu Chemicals, Inc.),<br>12 March, 1996 (12.03.96),<br>Example 74<br>& EP 688830 A1 & US 5639809 A1 | 1,4-7,11,12<br>2,3,8-10,<br>13-20 |
| X<br>A | JP 2-309302 A (Nippon Kayaku Co., Ltd.),<br>25 December, 1990 (25.12.90),<br>Example 5<br>(Family: none) | 4-7,11-17<br>1-3,8-10,<br>18-20 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>22 June, 2006 (22.06.06) | Date of mailing of the international search report<br>04 July, 2006 (04.07.06) |
| Name and mailing address of the ISA/<br>Japanese Patent Office<br>Facsimile No. | Authorized officer<br><br>Telephone No. |

Form PCT/ISA/210 (second sheet) (April 2005)

**INTERNATIONAL SEARCH REPORT**

International application No.
PCT/JP2006/307080

C (Continuation). DOCUMENTS CONSIDERED TO BE RELEVANT

| Category* | Citation of document, with indication, where appropriate, of the relevant passages | Relevant to claim No. |
|---|---|---|
| X<br>A | JP 6-128498 A (Mitsui Toatsu Chemicals, Inc.), 10 May, 1994 (10.05.94), Example 17 (Family: none) | 4-7,11,12<br>1-3,8-10, 13-20 |
| X<br>A | JP 5-295281 A (Mitsui Toatsu Chemicals, Inc.), 09 November, 1993 (09.11.93), Example 19, 21, 23, 26, 29, 31, 33, 36, 37, 39, 41, 43, 48, 50, 51, 52, 55 & EP 549342 A2 & US 5318856 A1 | 4-7,11,12<br>1-3,8-10, 13-20 |
| X<br>A | JP 5-295282 A (Mitsui Toatsu Chemicals, Inc.), 09 November, 1993 (09.11.93), Example 2, formula (VIII); example 5 (Family: none) | 4-7,11,12<br>1-3,8-10, 13-20 |
| X<br>A | JP 1-313568 A (Nippon Kayaku Co., Ltd.), 19 December, 1989 (19.12.89), Example 22, 25 | 4-7,11,12<br>1-3,8-10, 13-20 |
| X<br>A | JP 8-143435 A (Mitsubishi Chemical Corp.), 04 June, 1996 (04.06.96), Formulas [IV], [IX] (Family: none) | 1,3-7,11<br>2,8-10,12-20 |
| X<br>A | JP 61-285275 A (Canon Inc.), 16 December, 1986 (16.12.86), No.14 & US 4841037 A1 & GB 8617171 A | 1,3,11<br>2,4-10,12-20 |
| A | JP 1-193375 A (Canon Inc.), 03 August, 1989 (03.08.89), Claims (Family: none) | 1-20 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2002180052 A
- JP 2002528758 A
- JP 2002338838 A
- JP 8511109 A
- JP 2841377 B
- JP 3094113 B
- JP 3168850 B
- JP 2002169025 A
- JP 2003029030 A
- JP 2005107636 A
- JP 2005110535 A
- JP 2005123092 A
- JP 2005295499 A
- JP 2006084605 A


### Non-patent literature cited in the description


- **DREYER, J.F.** Light Polarization From Films of Lyotropic Nematic Liquid Crystals. *Journal de Physique,* 1969, vol. 4, 114
- **YUTAKA HOSODA.** Shin Senryo Kagaku (New Dye Chemical. GIHODO SHUPPAN Co., Ltd, 21 December 1973, 396-409
- **YUTAKA HOSODA.** Shin Senryo Kagaku (New Dye Chemical. GIHODO SHUPPAN Co., Ltd, 21 December 1973, 396-409
- New Color Science Handbook. UNIVERSITY OF TOKYO PRESS, 25 November 1989, 104-105
- **YUJI HARASAKI.** Coating Engineering. Asakura Shoten K.K, 20 March 1971, 253-277
- **KUNIHIRO ICHIMURA.** Creation and Applications of Harmonized Molecular Materials. CMC Publishing Co., Ltd, 03 March 1998, 118-149
- Ekisho Binran (Liquid Crystal Handbook. Maruzen Company, Limited, 30 October 2000, 226-239